(19) Europäisches Patentamt
European Patent Office
Office européen des brevets

(11) EP 2 051 976 B1

# (12) EUROPEAN PATENT SPECIFICATION

(45) Date of publication and mention of the grant of the patent:
**26.05.2010 Bulletin 2010/21**

(21) Application number: **07810278.7**

(22) Date of filing: **05.07.2007**

(51) Int Cl.:
***C07D 401/14*** *(2006.01)*

(86) International application number:
**PCT/US2007/015670**

(87) International publication number:
**WO 2008/005569 (10.01.2008 Gazette 2008/02)**

(54) **4-[5-METHOXY-6-(2-METHYL-6-[1,2,4]TRIAZOL-1-YL-PYRIDIN-3-YLAMINO)-PYRIMIDIN-4-YLOXY]-PIPERIDINE-1-CARBOXYLC ACID ISOPROPYL ESTER AS MODULATOR OF METABOLISM AND THE TREATMENT OF DISORDERS RELATED THERETO**

4-[5-METHOXY-6-(2-METHYL-6-[1,2,4]TRIAZOL-1-YL-PYRIDIN-3-YLAMINO)-PYRIMIDIN-4-YLOXY]-PIPERIDIN-1-CARBONSÄURE ISOPROPYL ESTER ALS STOFFWECHSELMODULATORE UND BEHANDLUNG VON DAMIT ASSOZIIERTEN ERKRANKUNGEN

4-[5-MÉTHOXY-6-(2-METHYL-6-[1,2,4]TRIAZOL-1-YL-PYRIDINE-3-YLAMINO)-PYRIMIDINE-4-YLOXY]-PIPERIDINE-1-ACIDE CARBOXYLIQUE ISOPROPYL ESTER EN TANT QUE MODULATEUR DU MÉTABOLISME, ET TRAITEMENT DE TROUBLES AU MOYEN DE CE MODULATEUR

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **06.07.2006 US 819278 P**

(43) Date of publication of application:
**29.04.2009 Bulletin 2009/18**

(73) Proprietor: **Arena Pharmaceuticals, Inc.**
**San Diego, CA 92121 (US)**

(72) Inventors:
- **JONES, Robert M.**
  **San Diego, California 92130 (US)**
- **LEHMANN, Juerg**
  **San Diego, California 92129 (US)**
- **SIU-TING WONG, Amy**
  **Poway, California 92064 (US)**

(74) Representative: **Wytenburg, Wilhelmus Johannes et al**
**Mewburn Ellis LLP**
**33 Gutter Lane**
**London**
**EC2V 8AS (GB)**

(56) References cited:
**WO-A-2005/007647 WO-A-2005/121121**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 051 976 B1

## Description

The present invention relates to 4-[5-Methoxy-6-(2-methyl-6-[1,2,4]triazol-1-yl-pyridin-3-yla1nino)-pyrinlidin-4-yloxy]-piperidine-1-carboxylic acid isopropyl ester, and pharmaceutically acceptable salts, solvates and hydrates thereof that are modulators of glucose metabolism. Accordingly, compounds of the present invention are useful in the treatment of metabolic-related disorders and complications thereof, such as, diabetes and obesity.

Diabetes mellitus is a serious disease afflicting over 100 million people worldwide. In the United States, there are more than 12 million diabetics, with 600,000 new cases diagnosed each year.

Diabetes mellitus is a diagnostic term for a group of disorders characterized by abnormal glucose homeostasis resulting in elevated blood sugar. There are many types of diabetes, but the two most common are Type I (also referred to as insulin-dependent diabetes mellitus or IDDM) and Type II (also referred to as non-insulin-dependent diabetes mellitus or NIDDM).

The etiology of the different types of diabetes is not the same; however, everyone with diabetes has two things in common: overproduction of glucose by the liver and little or no ability to move glucose out of the blood into the cells where it becomes the body's primary fuel.

People who do not have diabetes rely on insulin, a hormone made in the pancreas, to move glucose from the blood into the cells of the body. However, people who have diabetes either don't produce insulin or can't efficiently use the insulin they produce; therefore, they can't move glucose into their cells. Glucose accumulates in the blood creating a condition called hyperglycemia, and over time, can cause serious health problems.

Diabetes is a syndrome with interrelated metabolic, vascular, and neuropathic components. The metabolic syndrome, generally characterized by hyperglycemia, comprises alterations in carbohydrate, fat and protein metabolism caused by absent or markedly reduced insulin secretion and/or ineffective insulin action. The vascular syndrome consists of abnormalities in the blood vessels leading to cardiovascular, retinal and renal complications. Abnormalities in the peripheral and autonomic nervous systems are also part of the diabetic syndrome.

About 5% to 10% of the people who have diabetes have IDDM. These individuals don't produce insulin and therefore must inject insulin to keep their blood glucose levels normal. IDDM is characterized by low or undetectable levels of endogenous insulin production caused by destruction of the insulin-producing β cells of the pancreas, the characteristic that most readily distinguishes IDDM from NIDDM. IDDM, once termed juvenile-onset diabetes, strikes young and older adults alike.

Approximately 90 to 95% of people with diabetes have Type II (or NIDDM). NIDDM subjects produce insulin, but the cells in their bodies are insulin resistant: the cells don't respond properly to the hormone, so glucose accumulates in their blood. NIDDM is characterized by a relative disparity between endogenous insulin production and insulin requirements, leading to elevated blood glucose levels. In contrast to IDDM, there is always some endogenous insulin production in NIDDM; many NIDDM patients have normal or even elevated blood insulin levels, while other NIDDM patients have inadequate insulin production (Rotwein, R. et al. N. Engl. J. Med. 308, 65-71 (1983)). Most people diagnosed with NIDDM are age 30 or older, and half of all new cases are age 55 and older. Compared with whites and Asians, NIDDM is more common among Native Americans, African-Americans, Latinos, and Hispanics. In addition, the onset can be insidious or even clinically inapparent, making diagnosis difficult.

The primary pathogenic lesion on NIDDM has remained elusive. Many have suggested that primary insulin resistance of the peripheral tissues is the initial event. Genetic epidemiological studies have supported this view. Similarly, insulin secretion abnormalities have been argued as the primary defect in NIDDM. It is likely that both phenomena are important contributors to the disease process (Rimoin, D. L., et. al. Emery and Rimoin's Principles and Practice of Medical Genetics 3rd Ed. 1:1401-1402 (1996)).

Many people with NIDDM have sedentary lifestyles and are obese: they weigh approximately 20% more than the recommended weight for their height and build. Furthermore, obesity is characterized by hyperinsulinemia and insulin resistance, a feature shared with NIDDM, hypertension and atherosclerosis.

Obesity and diabetes are among the most common human health problems in industrialized societies. In industrialized countries a third of the population is at least 20% overweight. In the United States, the percentage of obese people has increased from 25% at the end of the 1970's, to 33% at the beginning the 1990's. Obesity is one of the most important risk factors for NIDDM. Definitions of obesity differ, but in general, a subject weighing at least 20% more than the recommended weight for his/her height and build is considered obese. The risk of developing NIDDM is tripled in subjects 30% overweight, and three-quarters with NIDDM are overweight.

Obesity, which is the result of an imbalance between caloric intake and energy expenditure, is highly correlated with insulin resistance and diabetes in experimental animals and human. However, the molecular mechanisms that are involved in obesity-diabetes syndromes are not clear. During early development of obesity, increased insulin secretion balances insulin resistance and protects patients from hyperglycemia (Le Stunff, et al. Diabetes 43, 696-702 (1989)). However, after several decades, β cell function deteriorates and non-insulin-dependent diabetes develops in about 20% of the obese population (Pederson, P. Diab. Metab. Rev. 5, 505-509 (1989)) and (Brancati, F. L., et al., Arch. Intern.

Med. 159, 957-963 (1999)). Given its high prevalence in modem societies, obesity has thus become the leading risk factor for NIDDM (Hill, J. O., et al., Science 280, 1371-1374 (1998)). However, the factors which predispose a fraction of patients to alteration of insulin secretion in response to fat accumulation remain unknown.

Whether someone is classified as overweight or obese is generally determined on the basis of their body mass index (BMI) which is calculated by dividing body weight (kg) by height squared ($m^2$). Thus, the units of BMI are $kg/m^2$ and it is possible to calculate the BMI range associated with minimum mortality in each decade of life. Overweight is defined as a BMI in the range 25-30 $kg/m^2$, and obesity as a BMI greater than 30 $kg/m^2$ (see TABLE below). There are problems with this definition in that it does not take into account the proportion of body mass that is muscle in relation to fat (adipose tissue). To account for this, obesity can also be defined on the basis of body fat content: greater than 25% and 30% in males and females, respectively.

**CLASSIFICATION OF WEIGHT BY BODY MASS INDEX (BMI)**

| BMI | CLASSIFICATION |
|---|---|
| < 18.5 | Underweight |
| 18.5-24.9 | Normal |
| 25.0-29.9 | Overweight |
| 30.0-34.9 | Obesity (Class I) |
| 35.0-39.9 | Obesity (Class II) |
| >40 | Extreme Obesity (Class III) |

As the BMI increases there is an increased risk of death from a variety of causes that is independent of other risk factors. The most common diseases with obesity are cardiovascular disease (particularly hypertension), diabetes (obesity aggravates the development of diabetes), gall bladder disease (particularly cancer) and diseases of reproduction. Research has shown that even a modest reduction in body weight can correspond to a significant reduction in the risk of developing coronary heart disease.

Obesity considerably increases the risk of developing cardiovascular diseases as well. Coronary insufficiency, atheromatous disease, and cardiac insufficiency are at the forefront of the cardiovascular complication induced by obesity. It is estimated that if the entire population had an ideal weight, the risk of coronary insufficiency would decrease by 25% and the risk of cardiac insufficiency and of cerebral vascular accidents by 35%. The incidence of coronary diseases is doubled in subjects less than 50 years of age who are 30% overweight. The diabetes patient faces a 30% reduced lifespan. After age 45, people with diabetes are about three times more likely than people without diabetes to have significant heart disease and up to five times more likely to have a stroke. These findings emphasize the inter-relations between risks factors for NIDDM and coronary heart disease and the potential value of an integrated approach to the prevention of these conditions (Perry, I. J., et al., BMJ 310, 560-564 (1995)).

Diabetes has also been implicated in the development of kidney disease, eye diseases and nervous-system problems. Kidney disease, also called nephropathy, occurs when the kidney's "filter mechanism" is damaged and protein leaks into urine in excessive amounts and eventually the kidney fails. Diabetes is also a leading cause of damage to the retina at the back of the eye and increases risk of cataracts and glaucoma. Finally, diabetes is associated with nerve damage, especially in the legs and feet, which interferes with the ability to sense pain and contributes to serious infections. Taken together, diabetes complications are one of the nation's leading causes of death.

WO 2005/007647 A1 (Arena Pharmaceuticals, Inc.) was published on 27 January 2005. The document describes certain trisubstituted aryl and heteroaryl derivatives of the following formula as modulators of metabolism for the treatment of metabolic-related disorders and complications thereof, such as diabetes and obesity.

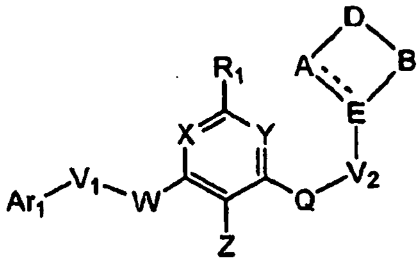

(I)

The following specific examples are described therein:

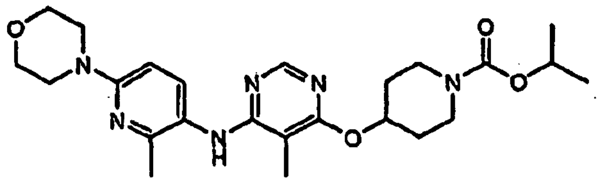

Compound A91

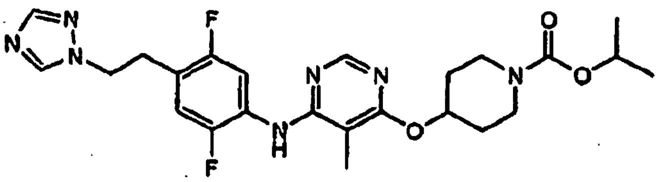

Compound A55

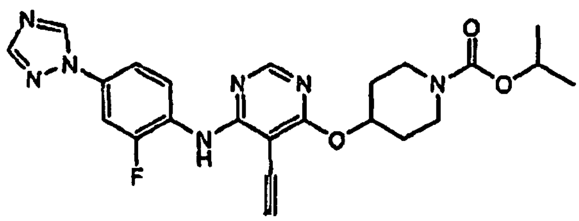

Compound A48

WO 2005/121121 A2 (Arena Pharmaceuticals, Inc.) was published on 22 December 2005. The document describes certain substituted aryl and heteroaryl derivatives of the following formula as modulators of metabolism for the treatment of metabolic-related disorders and complications thereof, such as diabetes and obesity.

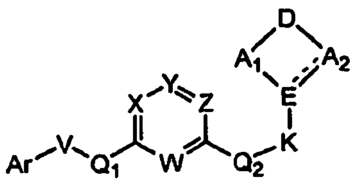

(I)

## SUMMARY OF THE INVENTION

The present invention relates to compounds which bind to and modulate the activity of a GPCR, referred to herein as **RUP3,** and uses thereof. The term **RUP3** as used herein includes the human sequences found in GeneBank

accession number AY288416, naturally-occurring allelic variants, mammalian orthologs, and recombinant mutants thereof. A preferred human **RUP3** for use in screening and testing of the compounds of the invention is provided in the nucleotide sequence of Seq. ID.No:1 and the corresponding amino acid sequence in Seq. IID.No:2 found in PCT publication No. WO2005/007647.

One aspect of the present invention pertains to the compound, 4-[5-Metboxy-6-(2-methyl-6-[1,2,4]triazol-1-yl-pyridin-3-ylamino)-pyrimidin-4-yloxy]-piperidine-1-carboxylic acid isopropyl ester, as shown in Formula **(I):**

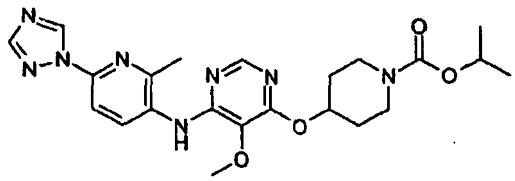

(I)

and pharmaceutically acceptable salts, solvates, and hydrates thereof.

One aspect of the present invention pertains to pharmaceutical compositions comprising a compound of the present invention and a pharmaceutically acceptable carrier.

Also described herein are methods of treating a metabolic-related disorder in an individual comprising administering to the individual in need of such treatment a therapeutically effective amount of a compound of the present invention or a pharmaceutical composition thereof.

Also described herein are methods of treating obesity in an individual comprising administering to the individual in need of such treatment a therapeutically effective amount of a compound of the present invention or a pharmaceutical composition thereof.

Also described herein are methods of decreasing food intake of an individual comprising administering to the individual in need thereof a therapeutically effective amount of a compound of the present invention or pharmaceutical composition thereof.

Also described herein are methods of inducing satiety in an individual comprising administering to the individual in need thereof a therapeutically effective amount of a compound of the present invention or pharmaceutical composition thereof.

Also described herein are methods of controlling or decreasing weight gain of an individual comprising administering to the individual in need thereof a therapeutically effective amount of a compound of the present invention or pharmaceutical composition thereof.

Also described herein are methods of modulating a **RUP3** receptor in an individual comprising contacting the receptor with a compound of the present invention. In some embodiments, the compound is an agonist for the **RUP3** receptor.

Also described herein are methods of modulating a **RUP3** receptor for the treatment of a metabolic-related disorder.

Also described herein are methods of modulating a **RUP3** receptor in an individual comprising contacting the receptor with a compound of the present invention wherein the modulation of the **RUP3** receptor reduces food intake of the individual.

Also described herein are methods of modulating a **RUP3** receptor in an individual comprising contacting the receptor with a compound of the present invention wherein the modulation of the **RUP3** receptor induces satiety in the individual.

Also described herein are methods of modulating a **RUP3** receptor in an individual comprising contacting the receptor with a compound of the present invention wherein the modulation of the **RUP3** receptor controls or reduces weight gain of the individual.

One aspect of the present invention pertains to the use of a compound of the present invention for production of a medicament for use in the treatment of a metabolic-related disorder.

One aspect of the present invention pertains to the use of a compound of the present invention for production of a medicament for use in decreasing food intake in an individual.

One aspect of the present invention pertains to the use of a compound of the present invention for production of a medicament for use of inducing satiety in an individual.

One aspect of the present invention pertains to the use of a compound of the present invention for production of a medicament for use in controlling or decreasing weight gain in an individual.

One aspect of the present invention pertains to a compound of the present invention for use in a method of treatment of the human or animal body by therapy.

One aspect of the present invention pertains to a compound of the present invention for use in a method of treatment of a metabolic-related disorder of the human or animal body by therapy.

In some embodiments the individual is a mammal. In some embodiments the mammal is a human.

Some embodiments of the present invention pertain to wherein the human has a body mass index of about 18.5 to about 45. In some embodiments, the human has a body mass index of about 25 to about 45. In some embodiments, the human has a body mass index of about 30 to about 45. In some embodiments, the human has a body mass index of about 35 to about 45.

In some embodiments, the metabolic-related disorder is type I diabetes, type II diabetes, inadequate glucose tolerance, insulin resistance, hyperglycemia, hyperlipidemia, hypertriglyceridemia, hypercholesterolemia, dyslipidemia or syndrome X. In some embodiments, the metabolic-related disorder is type II diabetes. In some embodiments, the metabolic-related disorder is hyperglycemia. In some embodiments, the metabolic-related disorder is hyperlipidemia. In some embodiments, the metabolic-related disorder is hypertriglyceridemia. In some embodiments, the metabolic-related disorder is type I diabetes. In some embodiments, the metabolic-related disorder is dyslipidemia. In some embodiments, the metabolic-related disorder is syndrome X.

One aspect of the present invention pertains to methods of preparing pharmaceutical compositions comprising admixing a compound of the present invention and a pharmaceutically acceptable carrier.

Applicant reserves the right to exclude any one or more of the compounds from any of the embodiments of the invention. Applicant additionally reserves the right to exclude any disease, condition or disorder from any of the embodiments of the invention.

## BRIEF DESCRIPTION OF THE DRAWINGS

**Figure 1** shows dose escalation pharmacokinetics AUC vs dose for 4-[5-Methoxy-6-(2-methyl-6-[1,2,4]triazol-1-yl-pyridin-3-ylamino)-pyrimidin-4-yloxy]-piperidine-1-carboxylic acid isopropyl ester (i.e., Compound of Formula **(I))** compared to different RUP3 compounds, see Example 5 for details.

## DETAILED DESCRIPTION OF THE INVENTION

The invention is described herein in detail using the terms defined below unless otherwise specified.

**AGONIST** shall mean a moiety that interact and activate the receptor, such as the **RUP3** receptor, and initiates a physiological or pharmacological response characteristic of that receptor. For example, when moieties activate the intracellular response upon binding to the receptor, or enhance GTP binding to membranes.

**COMPOSITION** shall mean a material comprising at least two compounds or two components; for example, and without limitation, a Pharmaceutical Composition is a Composition comprising a compound of the present invention and a pharmaceutically acceptable carrier.

**CONTACT** or **CONTACTING** shall mean bringing the indicated moieties together, whether in an in vitro system or an in vivo system. Thus, "contacting" a **RUP3** receptor with a compound of the invention includes the administration of a compound of the present invention to an individual, for example a human, having a **RUP3** receptor, as well as, for example, introducing a compound of the invention into a sample containing a cellular or more purified preparation containing a **RUP3** receptor.

**IN NEED OF TREATMENT** as used herein refers to a judgment made by a caregiver (e.g. physician, nurse, nurse practitioner, etc. in the case of humans; veterinarian in the case of animals, including non-human mammals) that an individual or animal requires or will benefit from treatment. This judgment is made based on a variety of factors that are in the realm of a caregiver's expertise, but that includes the knowledge that the individual is ill, or will be ill, as the result of a disease, condition or disorder that is treatable by the compounds of the invention. The term "treatment" also refers in the alternative to "prophylaxis." Therefore, in general, "in need of treatment" refers to the judgment of the caregiver that the individual is already ill, accordingly, the compounds of the present invention are used to alleviate, inhibit or ameliorate the disease, condition or disorder. Furthermore, the phrase also refers, in the alternative, to the judgment made by the caregiver that the individual will become ill. In this context, the compounds of the invention are used in a protective or preventive manner.

**INDIVIDUAL** as used herein refers to any animal, in one embodiment is a vertebrate, in another embodiment is a mammal (both non-primate and primate), and examples include but not limited to cow, horse, sheep, swine, chicken, turkey, quail, cat, dog, mouse, rat, rabbit, guinea pig, other rodent, monkey, and the like. In another embodiment, is a human and in certain embodiments, the human is an infant, child, adolescent or adult. In one embodiment, the patient is at risk for developing a metabolic-related disease or disorder. A Patient who is at risk include, but are not limited to, those with hereditary history of a metabolic-related disease or disorder, or is in a state of physical health which puts the

patient at risk for a metabolic-related disease or disorder. In another embodiment, the patient has been determined, by the care-giver or someone acting under the guidance of the care-giver, to have a metabolic-related disease or disorder.

**INHIBIT** or **INHIBITING,** in relationship to the term "response" shall mean that a response is decreased or prevented in the presence of a compound as opposed to in the absence of the compound.

As used herein, the terms **MODULATE** or **MODULATING** shall mean to refer to an increase or decrease in the amount, quality, response or effect of a particular activity, function or molecule.

**PHARMACEUTICAL COMPOSITION** shall mean a composition comprising at least one compound of the present invention and at least one pharmaceutically acceptable exceipient/carrier. Those of ordinary skill in the art will understand and appreciate the techniques appropriate for preparing such compositions.

**THERAPEUTICALLY EFFECTIVE AMOUNT** as used herein refers to the amount of active compound or pharmaceutical composition that elicits the biological or medicinal response in a tissue, system, animal, individual or human that is being sought by a researcher, veterinarian, medical doctor or other clinician, which includes one or more of the following:

(1) Preventing the disease; for example, preventing a disease, condition or disorder in an individual that may be predisposed to the disease, condition or disorder but does not yet experience or display the pathology or symptomatology of the disease,

(2) Inhibiting the disease; for example, inhibiting a disease, condition or disorder in an individual that is experiencing or displaying the pathology or symptomatology of the disease, condition or disorder (i.e., arresting further development of the pathology and/or symptomatology), and

(3) Ameliorating the disease; for example, ameliorating a disease, condition or disorder in an individual that is experiencing or displaying the pathology or symptomatology of the disease, condition or disorder (i.e., reversing/deminishing the pathology and/or symptomatology).

## COMPOUNDS OF THE PRESENT INVENTION

The compound 4-[5-Methoxy-6-(2-methyl-6-[1,2,4]triazol-1-yl-pyridin-3-ylamino)-pyrimidin-4-yloxy]-piperidine-1-carboxylic acid isopropyl ester, as shown in Formula **(I),** is a potent agonist of the **RUP3** receptor and is able to lower blood glucose in the oGTT model. Further, 4-[5-Methoxy-6-(2-methyl-6-[1,2,4]triazol-1-yl-pyridin-3-ylamino)-pyrimidin-4-yloxy]-piperidine-1-carboxylic acid isopropyl ester also demonstrates linear dose escalation pharmacokinetics.

The compound, 4-[5-Methoxy-6-(2-methyl-6-[1,2,4]triazol-1-yl-pyridin-3-ylamino)-pyrimidin-4-yloxy]-piperidine-1-carboxylic acid isopropyl ester, also exhibits improved characteristics in regard to cytochrome P450 enzymes.

Therefore, the present invention provides 4-[5-Methoxy-6-(2-methyl-6-[1,2,4]triazol-1-yl-pyridin-3-ylamino)-pyrimidin-4-yloxy]-piperidine-1-carboxylic acid isopropyl ester and methods for the treatment of **RUP3** receptor related disorders, for example, metabolic-related disorders and complications thereof, such as, diabetes and obesity.

One aspect of the present invention pertains to the compound, 4-[5-Methoxy-6-(2-methyl-6-[1,2,4]triazol-1-yl-pyridin-3-ylamino)-pyrimidin4-yloxy]-piperidine-1-carboxylic acid isopropyl ester, as shown in Formula **(I):**

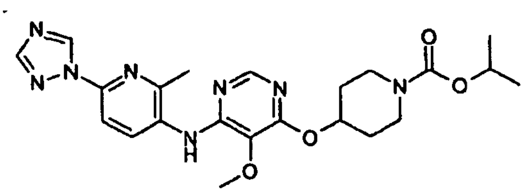

(I)

and pharmaceutically acceptable salts, solvates, and hydrates thereof.

## INDICATIONS AND METHODS OF TREATMENT

In addition to the foregoing beneficial uses for compounds of the present invention disclosed herein, compounds of the invention are useful in the treatment of additional diseases. Without limitation, these include the following.

The most significant pathologies in Type II diabetes are impaired insulin signaling at its target tissues ("insulin resistance") and failure of the insulin-producing cells of the pancreas to secrete an appropriate degree of insulin in response to a hyperglycemic signal. Current therapies to treat the latter include inhibitors of the β-cell ATP-sensitive potassium channel to trigger the release of endogenous insulin stores, or administration of exogenous insulin. Neither of these achieves accurate normalization of blood glucose levels and both carry the risk of inducing hypoglycemia. For

these reasons, there has been intense interest in the development of pharmaceuticals that function in a glucose-dependent action, i.e. potentiators of glucose signaling. Physiological signaling systems which function in this manner are well-characterized and include the gut peptides GLP1, GIP and PACAP. These hormones act via their cognate G-protein coupled receptor to stimulate the production of cAMP in pancreatic β-cells. The increased cAMP does not appear to result in stimulation of insulin release during the fasting or preprandial state. However, a series of biochemical targets of cAMP signaling, including the ATP-sensitive potassium channel, voltage-sensitive potassium channels and the exocytotic machinery, are modified in such a way that the insulin secretory response to a postprandial glucose stimulus is markedly enhanced. Accordingly, agonists of novel, similarly functioning, β-cell GPCRs, including **RUP3,** would also stimulate the release of endogenous insulin and consequently promote normoglycemia in Type II diabetes.

It is also established that increased cAMP, for example as a result of GLP1 stimulation, promotes β-cell proliferation, inhibits β-cell death and thus improves islet mass. This positive effect on β-cell mass is expected to be beneficial in both Type II diabetes, where insufficient insulin is produced, and Type I diabetes, where β-cells are destroyed by an inappropriate autoimmune response.

Some β-cell GPCRs, including **RUP3,** are also present in the hypothalamus where they modulate hunger, satiety, decrease food intake, controlling or decreasing weight and energy expenditure. Hence, given their function within the hypothalamic circuitry, agonists or inverse agonists of these receptors mitigate hunger, promote satiety and therefore modulate weight.

It is also well-established that metabolic diseases exert a negative influence on other physiological systems. Thus, there is often the codevelopment of multiple disease states (e.g. type I diabetes, type II diabetes, inadequate glucose tolerance, insulin resistance, hyperglycemia, hyperlipidemia, hypertriglyceridemia, hypercholesterolemia, dyslipidemia, obesity or cardiovascular disease in "Syndrome X") or secondary diseases which clearly occur secondary to diabetes (e.g. kidney disease, peripheral neuropathy). Thus, it is expected that effective treatment of the diabetic condition will in turn be of benefit to such interconnected disease states.

In some embodiments of the present invention the metabolic-related disorder is hyperlipidemia, type 1 diabetes, type 2 diabetes mellitus, idiopathic type 1 diabetes (Type 1b), latent autoimmune diabetes in adults (LADA), early-onset type 2 diabetes (EOD), youth-onset atypical diabetes (YOAD), maturity onset diabetes of the young (MODY), malnutrition-related diabetes, gestational diabetes, coronary heart disease, ischemic stroke, restenosis after angioplasty, peripheral vascular disease, intermittent claudication, myocardial infarction (e.g. necrosis and apoptosis), dyslipidemia, post-prandial lipemia, conditions of impaired glucose tolerance (IGT), conditions of impaired fasting plasma glucose, metabolic acidosis, ketosis, arthritis, obesity, osteoporosis, hypertension, congestive heart failure, left ventricular hypertrophy, peripheral arterial disease, diabetic retinopathy, macular degeneration, cataract, diabetic nephropathy, glomerulosclerosis, chronic renal failure, diabetic neuropathy, metabolic syndrome, syndrome X, premenstrual syndrome, coronary heart disease, angina pectoris, thrombosis, atherosclerosis, myocardial infarction, transient ischemic attacks, stroke, vascular restenosis, hyperglycemia, hyperinsulinemia, hyperlipidemia, hypertrygliceridemia, insulin resistance, impaired glucose metabolism, conditions of impaired glucose tolerance, conditions of impaired fasting plasma glucose, obesity, erectile dysfunction, skin and connective tissue disorders, foot ulcerations and ulcerative colitis, endothelial dysfunction and impaired vascular compliance.

## PHARMACEUTICAL COMPOSITIONS AND SALTS

A further aspect of the present invention pertains to pharmaceutical compositions comprising 4-[5-Methoxy-6-(2-methyl-6-[1,2,4]triazol-1-yl-pyridin-3-ylamino)-pyrimidin-4-yloxy]-piperidine-1-carboxylic acid isopropyl ester, Formula **(I),** a pharmaceutically acceptable salt, solvate or hydrate thereof and one or more pharmaceutically acceptable carriers. Some embodiments of the present invention pertain to pharmaceutical compositions comprising 4-[5-Methoxy-6-(2-methyl-6-[1,2,4]triazol-1-yl-pyridin-3-ylamino)-pyrimidin-4-yloxy]-piperidine-1-carboxylic acid isopropyl ester and a pharmaceutically acceptable carrier.

Some embodiments of the present invention include a method of producing a pharmaceutical composition comprising admixing 4-[5-Methoxy-6-(2-methyl-6-[1,2,4]triazol-1-yl-pyridin-3-ylamino)-pyrimidin-4.-yloxy]-piperidine-1-carboxylic acid isopropyl ester or a pharmaceutically acceptable salt thereof and a pharmaceutically acceptable carrier.

Formulations may be prepared by any suitable method, typically by uniformly mixing the active compound with liquids or finely divided solid carriers, or both, in the required proportions, and then, if necessary, forming the resulting mixture into a desired shape.

Conventional excipients, such as binding agents, fillers, acceptable wetting agents, tabletting lubricants, and disintegrants may be used in tablets and capsules for oral administration. Liquid preparations for oral administration may be in the form of solutions, emulsions, aqueous or oily suspensions, and syrups. Alternatively, the oral preparations may be in the form of dry powder that can be reconstituted with water or another suitable liquid vehicle before use. Additional additives such as suspending or emulsifying agents, non-aqueous vehicles (including edible oils), preservatives, and flavorings and colorants may be added to the liquid preparations. Parenteral dosage forms may be prepared

by dissolving the compound of the invention in a suitable liquid vehicle and filter sterilizing the solution before filling and sealing an appropriate vial or ampoule. These are just a few examples of the many appropriate methods well known in the art for preparing dosage forms.

A compound of the present invention can be formulated into pharmaceutical compositions using techniques well known to those in the art. Suitable pharmaceutically-acceptable carriers, outside those mentioned herein, are known in the art; for example, see Remington, The Science and Practice of Pharmacy, 20th Edition, 2000, Lippincott Williams & Wilkins, (Editors: Gennaro, A. R., et al.).

While it is possible that, for use in the treatment, a compound of the invention may, in an alternative use, be administered as a raw or pure chemical, it is preferable however to present the compound or active ingredient as a pharmaceutical formulation or composition further comprising a pharmaceutically acceptable carrier.

The invention thus further provides pharmaceutical formulations comprising a compound of the invention or a pharmaceutically acceptable salt or derivative thereof together with one or more pharmaceutically acceptable carriers thereof and/or prophylactic ingredients. The carrier(s) must be "acceptable" in the sense of being compatible with the other ingredients of the formulation and not overly deleterious to the recipient thereof.

Pharmaceutical formulations include those suitable for oral, rectal, nasal, topical (including buccal and sub-lingual), vaginal or parenteral (including intramuscular, sub-cutaneous and intravenous) administration or in a form suitable for administration by inhalation, insufflation or by a transdermal patch. Transdermal patches dispense a drug at a controlled rate by presenting the drug for absorption in an efficient manner with a minimum of degradation of the drug. Typically, transdermal patches comprise an impermeable backing layer, a single pressure sensitive adhesive and a removable protective layer with a release liner. One of ordinary skill in the art will understand and appreciate the techniques appropriate for manufacturing a desired efficacious transdermal patch based upon the needs of the artisan.

The compounds of the invention, together with a conventional adjuvant, carrier, or diluent, may thus be placed into the form of pharmaceutical formulations and unit dosages thereof, and in such form may be employed as solids, such as tablets or filled capsules, or liquids such as solutions, suspensions, emulsions, elixirs, gels or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units arc capsules, tablets, powders, granules or a suspension, with conventional additives such as lactose, mannitol, corn starch or potato starch; with binders such as crystalline cellulose, cellulose derivatives, acacia, corn starch or gelatins; with disintegrators such as corn starch, potato starch or sodium carboxymethyl-cellulose; and with lubricants such as talc or magnesium stearate. The active ingredient may also be administered by injection as a composition wherein, for example, saline, dextrose or water may be used as a suitable pharmaceutically acceptable carrier.

The dose when using the compounds of the present invention can vary within wide limits, and as is customary and is known to the physician, it is to be tailored to the individual conditions in each individual case. It depends, for example, on the nature and severity of the illness to be treated, on the condition of the patient, on the compound employed or on whether an acute or chronic disease state is treated or prophylaxis is conducted or on whether further active compounds are administered in addition to the compounds of the present invention. For example, doses of the present invention include, but not limited to, about 0.001 mg to about 5000 mg, about 0.001 to about 2500 mg, about 0.001 to about 1000 mg, 0.001 to about 500 mg, 0.001 mg to about 250 mg, about 0.001 mg to 100 mg, about 0.001 mg to about 50 mg, and about 0.001 mg to about 25 mg. The desired dose may conveniently be presented in a single dose or as divided doses administered at appropriate intervals, for example, as two, three, four or more sub-doses per day. The sub-dose itself may be further divided, e.g., into a number of discrete loosely spaced administrations. Depending on the individual and as deemed appropriate from the patient's physician or care-giver it may be necessary to deviate upward or downward from the doses described herein.

The amount of a compound of the invention, or pharmaceutically acceptable salt thereof, required for use in treatment will vary not only with the particular salt selected but also with the route of administration, the nature of the condition being treated and the age and condition of the patient and will ultimately be at the discretion of the attendant physician or clinician. In general, one skilled in the art understands how to extrapolate in vivo data obtained in a model system, typically an animal model, to another, such as a human. Typically, animal models include, but are not limited to, the rodent diabetes model as described in Example **1**, *infra* (as well as other animal models known in the art, such as those reported by Reed and Scribner in Diabetes, Obesity and Metabolism, 1, 1999, 75-86). In some circumstances, these extrapolations may merely be based on the weight of the animal in the respective model in comparison to another, such as a mammal, preferably a human, however, more often, these extrapolations are not simply based on weights,

but rather incorporate a variety of factors. Representative factors include, but not limited to, the type, age, weight, sex, diet and medical condition of the patient, the severity of the disease, the route of administration, pharmacological considerations such as the activity, efficacy, pharmacokinetic and toxicology profiles of the particular compound employed, whether a drug delivery system is utilized, on whether an acute or chronic disease state is being treated or prophylaxis is conducted or on whether further active compounds are administered in addition to the compounds of the present invention and as part of a drug combination. The dosage regimen for treating a disease condition with the compounds and/or compositions of this invention is selected in accordance with a variety factors as cited above. Thus, the actual dosage regimen employed may vary widely and therefore may deviate from a preferred dosage regimen and one skilled in the art will recognize that dosage and dosage regimen outside these typical ranges can be tested and, where appropriate, may be used in the methods of this invention.

The compounds of the present invention can be administrated in a wide variety of oral and parenteral dosage forms. It will be obvious to those skilled in the art that the following dosage forms may comprise, as the active component, either a compound of the invention or a pharmaceutically acceptable salt of a compound of the invention.

For preparing pharmaceutical compositions from the compounds of the present invention, the selection of a suitable pharmaceutically acceptable carrier can be either solid, liquid or a mixture of both. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid which is in a mixture with the finely divided active component.

In tablets, the active component is mixed with the carrier having the necessary binding capacity in suitable proportions and compacted to the desire shape and size.

The powders and tablets may contain varying percentage amounts of the active compound. A representative amount in a powder or tablet may contain from 0.5 to about 90 percent of the compound of the invention; however, an artisan would know when amounts outside of this range are necessary. Suitable carriers for powders and tablets are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as an admixture of fatty acid glycerides or cocoa butter, is first melted and the active component is dispersed homogeneously therein, as by stirring. The molten homogenous mixture is then poured into convenient sized molds, allowed to cool, and thereby to solidify.

Formulations suitable for vaginal administration may be presented as pessaries, tampons, creams, gels, pastes, foams or sprays containing in addition to the active ingredient such carriers as are known in the art to be appropriate.

Liquid form preparations include solutions, suspensions, and emulsions, for example, water or water-propylene glycol solutions. For example, parenteral injection liquid preparations can be formulated as solutions in aqueous polyethylene glycol solution. Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The pharmaceutical compositions may take such forms as suspensions, solutions, or emulsions in oily or aqueous vehicles, and may contain formulatory agents such as suspending, stabilizing and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous formulations suitable for oral use can be prepared by dissolving or suspending the active component in water and adding suitable colorants, flavours, stabilizing and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations

may contain, in addition to the active component, colorants, flavors, stabilizers, buffers, artificial and natural sweeteners, dispersants, thickeners, solubilizing agents, and the like.

For topical administration to the epidermis the compounds according to the invention may be formulated as ointments, creams or lotions, or as a transdermal patch.

Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents.

Formulations suitable for topical administration in the mouth include lozenges comprising active agent in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier.

Solutions or suspensions are applied directly to the nasal cavity by conventional means, for example with a dropper, pipette or spray. The formulations may be provided in single or multi-dose form. In the latter case of a dropper or pipette, this may be achieved by the patient administering an appropriate, predetermined volume of the solution or suspension. In the case of a spray, this may be achieved for example by means of a metering atomizing spray pump.

Administration to the respiratory tract may also be achieved by means of an aerosol formulation in which the active ingredient is provided in a pressurized pack with a suitable propellant. If the compounds of the present invention or pharmaceutical compositions comprising them are administered as aerosols, for example as nasal aerosols or by inhalation, this can be carried out, for example, using a spray, a nebulizer, a pump nebulizer, an inhalation apparatus, a metered inhaler or a dry powder inhaler. Pharmaceutical forms for administration of the compounds of the present invention as an aerosol can be prepared by processes well-known to the person skilled in the art. For their preparation, for example, solutions or dispersions of the compounds of the present invention in water, water/alcohol mixtures or suitable saline solutions can be employed using customary additives, for example benzyl alcohol or other suitable preservatives, absorption enhancers for increasing the bioavailability, solubilizers, dispersants and others, and, if appropriate, customary propellants, for example include carbon dioxide, CFC's, such as, dichlorodifluoromethane, trichlorofluoromethane, or dichlorotetrafluoroethane; and the like. The aerosol may conveniently also contain a surfactant such as lecithin. The dose of drug may be controlled by provision of a metered valve.

In formulations intended for administration to the respiratory tract, including intranasal formulations, the compound will generally have a small particle size for example of the order of 10 microns or less. Such a particle size may be obtained by means known in the art, for example by micronization. When desired, formulations adapted to give sustained release of the active ingredient may be employed.

Alternatively the active ingredients may be provided in the form of a dry powder, for example, a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

The pharmaceutical preparations are preferably in unit dosage forms. In such form, the preparation can be subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration are preferred compositions.

The compounds according to the invention may optionally exist as pharmaceutically acceptable salts including pharmaceutically acceptable acid addition salts prepared from pharmaceutically acceptable non-toxic acids including inorganic and organic acids.

The acid addition salts may be obtained as the direct product of compound synthesis. In the alternative, the free base may be dissolved in a suitable solvent .containing the appropriate acid, and the salt isolated by evaporating the solvent or otherwise separating the salt and solvent. The compounds of this invention may form solvates with standard low molecular weight solvents using methods known to the skilled artisan.

Some embodiments of the present invention include a method of producing a pharmaceutical composition for "combination-therapy" comprising admixing at least one compound of the present invention together with at least one pharmaceutical agent as described herein and together with a pharmaceutically acceptable carrier.

In some embodiments the pharmaceutical agents is selected from the group consisting of: sulfonylureas, meglitinides, biguanides, $\alpha$-glucosidase inhibitors, peroxisome proliferators-activated receptor-$\gamma$ (i.e., PPAR-$\gamma$) agonists, insulin, insulin analogues, HMG-CoA reductase inhibitors, cholesterol-lowering drugs (for example, fibrates that include: fenofibrate, bezafibrate, gemfibrozil, clofibrate and the like; bile acid sequestrants which include: cholestyramine, colestipol and the like; and niacin), antiplatelet agents (for example, aspirin and adenosine diphosphate receptor antagonists that include: clopidogrel, ticlopidine and the like), angiotensin-converting enzyme inhibitors, angiotensin II receptor antagonists and adiponectin.

It is noted that when the **RUP3** receptor modulators are utilized as active ingredients in a pharmaceutical composition, these are not intended for use only in humans, but in other non-human mammals as well. Indeed, recent advances in the area of animal health-care indicate that consideration be given for the use of active agents, such as **RUP3** receptor modulators, for the treatment of obesity in domestic animals (*e.g.*, cats and dogs), and **RUP3** receptor modulators in other domestic animals where no disease or disorder is evident (*e.g.*, food-oriented animals such as cows, chickens, fish, etc.). Those of ordinary skill in the art are readily credited with understanding the utility of such compounds in such settings.

## COMBINATION THERAPY

In the context of the present invention, a compound as described herein or pharmaceutical composition thereof can be utilized for modulating the activity of **RUP3** receptor mediated diseases, conditions and/or disorders as described herein. Examples of modulating the activity of **RUP3** receptor mediated diseases include the treatment of metabolic related disorders. Metabolic related disorders includes, but not limited to, hyperlipidemia, type 1 diabetes, type 2 diabetes mellitus, and conditions associated therewith, such as, but not limited to coronary heart disease, ischemic stroke, restenosis after angioplasty, peripheral vascular disease, intermittent claudication, myocardial infarction (e.g. necrosis and apoptosis), dyslipidemia, post-prandial lipemia, conditions of impaired glucose tolerance (IGT), conditions of impaired fasting plasma glucose, metabolic acidosis, ketosis, arthritis, obesity, osteoporosis, hypertension, congestive heart failure, left ventricular hypertrophy, peripheral arterial disease, diabetic retinopathy, macular degeneration, cataract, diabetic nephropathy, glomerulosclerosis, chronic renal failure, diabetic neuropathy, metabolic syndrome, syndrome X, premenstrual syndrome, coronary heart disease, angina pectoris, thrombosis, atherosclerosis, myocardial infarction, transient ischemic attacks, stroke, vascular restenosis, hyperglycemia, hyperinsulinemia, hyperlipidemia, hyperhygliceridemia, insulin resistance, impaired glucose metabolism, conditions of impaired glucose tolerance, conditions of impaired fasting plasma glucose, obesity, erectile dysfunction, skin and connective tissue disorders, foot ulcerations and ulcerative colitis, endothelial dysfunction and impaired vascular compliance. In some embodiments, metabolic related disorders include type I diabetes, type II diabetes, inadequate glucose tolerance, insulin resistance, hyperglycemia, hyperlipidemia, hypertriglyceridemia, hypercholesterolemia, dyslipidemia and syndrome X. Other examples of modulating the activity of **RUP3** receptor mediated diseases include the treatment of obesity and/or overweight by decreasing food intake, inducing satiation (i.e., the feeling of fullness), controlling weight gain, decreasing body weight and/or affecting metabolism such that the recipient loses weight and/or maintains weight.

While the compounds of the invention can be administered as the sole active pharmaceutical agent (i.e., monotherapy), they can also be used in combination with other pharmaceutical agents (i.e., combination-therapy) for the treatment of the diseases/conditions/disorders described herein. Therefore, another aspect of the present invention includes methods of treatment of a metabolic related disorder, including a weight related disorder, such as obesity, comprising administering to an individual in need of prophylaxis and/or treatment a therapeutically effective amount of a compound of the present invention in combination with one or more additional pharmaceutical agent as described herein.

Suitable pharmaceutical agents that can be used in combination with the compounds of the present invention include anti-obesity agents such as apolipoprotein-B secretion/microsomal triglyceride transfer protein (apo-B/MTP) inhibitors, MCR-4 agonists, cholescystokinin-A (CCK-A) agonists, serotonin and norepinephrine reuptake inhibitors (for example, sibutramine), sympathomimetic agents, β3 adrenergic receptor agonists, dopamine agonists (for example, bromocriptine), melanocyte-stimulating hormone receptor analogs, cannabinoid 1 receptor antagonists [for example, SR141716: N-(piperidin-1-yl)-5-(4-chlorophenyl)-1-(2,4-dichlorophenyl)-4-methyl-1H-pyrazole-3-carboxamide], melanin concentrating hormone antagonists, leptons (the OB protein), leptin analogues, leptin receptor agonists, galanin antagonists, lipase inhibitors (such as tetrahydrolipstatin, i.e., Orlistat), anorectic agents (such as a bombesin agonist), Neuropeptide-Y antagonists, thyromimetic agents, dehydroepiandrosterone or an analogue thereof, glucocorticoid receptor agonists or antagonists, orexin receptor antagonists, urocortin binding protein antagonists, glucagon-like peptide-1 receptor agonists, ciliary neutrotrophic factors (such as Axokine™ available from Regeneron Pharmaceuticals, Inc., Tarrytown, NY and Procter & Gamble Company, Cincinnati, OH), human agouti-related proteins (AGRP), ghrelin receptor antagonists, histamine 3 receptor antagonists or reverse agonists, neuromedin U receptor agonists, noradrenergic anorectic agents (for example, phentermine, mazindol and the like) and appetite suppressants (for example, bupropion).

Other anti-obesity agents, including the agents set forth *infra,* are well known, or will be readily apparent in light of the instant disclosure, to one of ordinary skill in the art.

In some embodiments, the anti-obesity agents are selected from the group consisting of orlistat, sibutramine, bromocriptine, ephedrine, leptin, and pseudoephedrine. In a further embodiment, compounds of the present invention and combination therapies are administered in conjunction with exercise and/or a sensible diet.

It is understood that the scope of combination-therapy of the compounds of the present invention with other

anti-obesity agents, anorectic agents, appetite suppressant and related agents is not limited to those listed above, but includes in principle any combination with any pharmaceutical agent or pharmaceutical composition useful for the treatment of overweight and obese individuals.

It is understood that the scope of combination-therapy of the compounds of the present invention with other pharmaceutical agents is not limited to those listed herein, *supra* or *infra,* but includes in principle any combination with any pharmaceutical agent or pharmaceutical composition useful for the treatment of diseases, conditions or disorders that are linked to metabolic related disorders.

Some embodiments of the present invention include methods of treatment of a disease, disorder, condition or complication thereof as described herein, comprising administering to an individual in need of such treatment a therapeutically effective amount or dose of a compound of the present invention in combination with at least one pharmaceutical agent selected from the group consisting of: sulfonylureas (for example, glyburide, glipizide, glimepiride and other sulfonylureas known in the art), mcglitinides (for example, repaglinide, nateglinide and other meglitinides known in the art), biguanides (for example, biguanides include phenformin, metformin, buformin, and biguanidcs known in the art), α-glucosidase inhibitors [for example, acarbose, N-(1,3-dihydroxy-2-propyl)valiolamine (generic name; voglibose), miglitol, and α-glucosidase inhibitors known in the art], peroxisome proliferators-activated receptor-y (i.e., PPAR-γ) agonists (for example, rosiglitazone, pioglitazone, tesaglitazar, netoglitazone, GW-409544, GW-501516 and PPAR-γ agonists known in the art), insulin, insulin analogues, HMG-CoA reductase inhibitors (for example, rosuvastatin, pravastatin and its sodium salt, simvastatin, lovastatin, atorvastatin, fluvastatin, cerivastatin, rosuvastatin, pitavastatin, BMS's "superstatin", and HMG-CoA reductase inhibitors known in the art), cholesterol-lowering drugs (for example, fibrates that include: bezafibrate, beclobrate, binifibrate, ciplofibrate, clinofibrate, clofibrate, clofibric acid, etofibrate, fenofibrate, gemfibrozil, nicofibrate, pirifibrate, ronifibrate, simfibrate, theofibrate, and fibrates known in the art; bile acid sequestrants which include: cholestyramine, colestipol and the like; and niacin), antiplatelet agents (for example, aspirin and adenosine diphosphate receptor antagonists that include: clopidogrel, ticlopidine and the like), angiotensin-converting enzyme inhibitors (for example, captopril, enalapril, alacepril, delapril; ramipril, lisinopril, imidapril, benazepril, ceronapril, cilazapril, enalaprilat, fosinopril, moveltopril, perindopril, quinapril, spirapril, temocapril, trandolapril, and angiotensin converting enzyme inhibitors known in the art), angiotensin II receptor antagonists [for example, losartan (and the potassium salt form)], angiotensin II receptor antagonists known in the art, adiponectin, squalene synthesis inhibitors {for example, (S)-α-[bis[2,2-dimethyl-1-oxopropoxy)methoxy] phosphinyl]-3-phenoxybenzenebutanesulfonic acid, mono potassium salt (BMS-188494) and squalene synthesis inhibitors known in the art}, and the like. In some embodiments, methods of the present invention include compounds of the present invention and the pharmaceutical agents are administered separately. In further embodiments, compounds of the present invention and the pharmaceutical agents are administered together.

Suitable pharmaceutical agents that can be used in conjunction with compounds of the present invention include, but not limited to, amylin agonists (for example, pramlintide), insulin secretagogues (for example, GLP-1 agonists; exendin-4; insulinotropin (NN2211); dipeptyl peptidase inhibitors (for example, NVP-DPP-728), acyl CoA cholesterol acetyltransferase inhibitors (for example, Ezetimibe, eflucimibe, and like compounds), cholesterol absorption inhibitors (for example, ezetimibe, pamaqueside and like compounds), cholesterol ester transfer protein inhibitors (for example, CP-529414, JTT-705, CETi-1, and like compounds), microsomal triglyceride transfer protein inhibitors (for example, implitapide, and like compounds), cholesterol modulators (for example, NO-1886, and like compounds), bile acid modulators (for example, GT103-279 and like compounds), insulin signalling pathway modulators, like inhibitors of protein tyrosine phosphatases (PTPases), non-small mol. mimetic compds. and inhibitors of glutamine-fhictose-6-phosphate amidotransferase (GFAT), compds. influencing a dysregulated hepatic glucose prodn., like inhibitors of glucose-6-phosphatase (G6Pase), inhibitors of fructose-1,6-bisphosphatase (F-1,6-BPase), inhibitors of glycogen phosphorylase (GP), glucagon receptor antagonists and inhibitors of phosphoenolpyruvate carboxykinase (PEPCK), pyruvate dehydrogenase kinase (PDHK) inhibitors, insulin sensitivity enhancers, insulin secretion enhancers, inhibitors of gastric emptying, $\alpha_2$-adrenergic antagonists and retinoid X receptor (RXR) agonists.

In accordance with the present invention, the combination can be used by mixing the respective active components, a compound of the present invention and pharmaceutical agent, either all together or independently with a physiologically acceptable carrier, excipient, binder, diluent, etc., as described herein above, and administering the mixture or mixtures either orally or non-orally as a pharmaceutical composition. When a compound or a mixture of compounds of the present invention are administered as a combination therapy with another active compound the therapeutic agents can be formulated as a separate pharmaceutical compositions given at the same time or at different times, or the therapeutic agents can be given as a single composition.

## OTHER UTILITIES

Another object of the present invention relates to radio-labeled compounds that would be useful not only in radio-imaging but also in assays, both in vitro and in vivo, for localizing and quantitating the **RUP3** receptor in tissue

samples, including human, and for identifying **RUP3** receptor ligands by inhibition binding of a radio-labeled compound. It is a further object of this invention to develop novel **RUP3** receptor assays of which comprise such radio-labeled compounds.

The present invention embraces isotopically-labeled compound of Formula **(I)** pharmaceutically acceptable salts thereof. An "isotopically" or "radio-labeled" compounds are those which are identical to compounds disclosed herein, but for the fact that one or more atoms are replaced or substituted by an atom having an atomic mass or mass number different from the atomic mass or mass number typically found in nature (i.e., naturally occurring). Suitable radionuclides that may be incorporated in compounds of the present invention include but are not limited to $^{2}H$ (also written as D for deuterium), $^{3}H$ (also written as T for tritium), $^{11}C$, $^{13}C$, $^{14}C$, $^{13}N$, $^{15}N$, $^{15}O$, $^{17}O$, $^{18}O$, $^{18}F$, $^{3S}S$, $^{36}Cl$, $^{82}Br$, $^{75}Br$, $^{76}Br$ , $^{77}Br$, $^{123}I$, $^{124}I$, $^{125}I$ and $^{131}I$. The radionuclide that is incorporated in the instant radio-labeled compounds will depend on the specific application of that radio-labeled compound. For example, for in vitro **RUP3** receptor labeling and competition assays, compounds that incorporate $^{3}H$, $^{14}C$, $^{82}Br$, $^{125}I$, $^{131}I$, $^{35}S$ or will generally be most useful. For radio-imaging applications $^{11}C$, $^{18}F$, $^{125}I$, $^{123}I$, $^{124}I$, $^{131}I$, $^{75}Br$, $^{76}Br$ or $^{77}Br$ will generally be most useful.

It is understood that a "radio-labeled " or "labeled compound" is a compound of present invention that has incorporated at least one radionuclide; in some embodiments the radionuclide is selected from the group consisting of $^{3}H$, $^{14}C$, $^{125}I$, $^{35}S$ and $^{82}Br$.

Certain isotopically-labeled compounds of the present invention are useful in compound and/or substrate tissue distribution assays. In some embodiments the radionuclide $^{3}H$ and/or $^{14}C$ isotopes are useful in these studies. Further, substitution with heavier isotopes such as deuterium (i.e., $^{2}H$) may afford certain therapeutic advantages resulting from greater metabolic stability (e.g., increased in vivo half-life or reduced dosage requirements) and hence may be preferred in some circumstances. Isotopically labeled compounds of the present invention can generally be prepared by following procedures analogous to those disclosed in the Schemes supra and Examples infra, by substituting an isotopically labeled reagent for a non-isotopically labeled reagent. Other synthetic methods that are useful are discussed *infra.* Moreover, it should be understood that all of the atoms represented in the compounds of the invention can be either the most commonly occurring isotope of such atoms or the more scarce radio-isotope or nonradio-active isotope.

Synthetic methods for incorporating radio-isotopes into organic compounds are applicable to compounds of the invention and are well known in the art. These synthetic methods can be used for an intermediate or the final compound, for example, incorporating activity levels of tritium into compounds of the present invention, are as follows:

A. Catalytic Reduction with Tritium Gas - This procedure normally yields high specific activity products and requires halogenated or unsaturated precursors.

B. Reduction with Sodium Borohydride [$^{3}H$] - This procedure is rather inexpensive and requires precursors containing reducible functional groups such as aldehydes, ketones, lactones, esters, and the like.

C. Reduction with Lithium Aluminum Hydride [$^{3}H$] - This procedure offers products at almost theoretical specific activities. It also requires precursors containing reducible functional groups such as aldehydes, ketones, lactones, esters, and the like.

D. Tritium Gas Exposure Labeling - This procedure involves exposing precursors containing exchangeable protons to tritium gas in the presence of a suitable catalyst.

E. N-Methylation using Methyl Iodide [$^{3}H$] - This procedure is usually employed to prepare O-methyl or N-methyl ($^{3}H$) products by treating appropriate precursors with high specific activity methyl iodide ($^{3}H$). This method in general allows for higher specific activity, such as for example, about 70-90 Ci/mmol.

A radio-labeled **RUP3** receptor compound of present invention can be used in a screening assay to identify/evaluate compounds. In general terms, a newly synthesized or identified compound (i.e., test compound) can be evaluated for its ability to reduce binding of the "radio-labeled compound" of the present invention to the **RUP3** receptor. Accordingly, the ability of a test compound to compete with the "radio-labeled compound" of the present invention for the binding to the **RUP3** receptor directly correlates to its binding affinity.

The labeled compounds of the present invention bind to the **RUP3** receptor. In one embodiment the labeled compound has an $IC_{50}$ less than about 500 $\mu$M, in another embodiment the labeled compound has an $IC_{50}$ less than about 100 $\mu$M, in yet another embodiment the labeled compound has an $IC_{50}$ less than about 10 $\mu$M, in yet another embodiment the labeled compound has an $IC_{50}$ less than about 1 $\mu$M, in still yet another embodiment the labeled inhibitor has an $IC_{50}$ less than about 0.1 $\mu$M, in still yet another embodiment the labeled inhibitor has an $IC_{50}$ less than about 0.01 $\mu$M, and in still yet another embodiment the labeled inhibitor has an $IC_{50}$ less than about 0.001 $\mu$M.

As will be recognized, the steps of the methods of the present invention need not be performed any particular number of times or in any particular sequence. Additional objects, advantages, and novel features of this invention will become apparent to those skilled in the art upon examination of the following examples thereof, which are intended to be illustrative and not intended to be limiting.

## EXAMPLES

### Example 1: In vivo effects of a RUP3 Agonist on glucose homeostasis in rats,

### General Procedure - Oral Glucose tolerance test (oGTT)

Male Sprague Dawley rats (Harlan, San Diego, CA) weighing approximately 350-375g were fasted for 16 hours and randomly grouped (n=6) to receive a RUP3 agonist at 0.3, 3 or 30 mg/kg. Compounds were delivered orally via a gavage needle (p.o., volume 2mL/kg). At time 0, levels of blood glucose were assessed using a glucometer (Accu-Chek Advantage, Roche Diagnostics), and rats were administered either vehicle (20% hydroxypropyl-beta-cyclodextrin) or test compound. Thirty minutes after administration of test compound, levels of blood glucose were again assessed, and rats were administered dextrose orally at a dose of 3g/kg. Blood glucose measurements were then taken 30 min, 60 min, and 120 min after this time. The **RUP3** agonist, 4-[5-Methoxy-6-(2-methyl-6-[1,2,4]thazol-1-yl-pyridin-3-ylamino)-pyrimidin-4-yloxy]-piperidine-1-carboxylic acid isopropyl ester [Formula (**I**)], showed a 60% mean percent inhibition of glucose excursion, averaged across the six animals in the treatment group. This demonstrates that the **RUP3** agonist, 4-[5-Methoxy-6-(2-methyl-6-[1,2,4]triazol-1-yl-pyridin-3-ylamino)-pyrimidin-4-yloxy]-piperidine-1-carboxylic acid isopropyl ester, lowered blood glucose after a challenge with glucose.

### Example 2: Receptor Binding Assay

In addition to the methods described herein, another means for evaluating a test compound is by determining binding affinities to the **RUP3** receptor. This type of assay generally requires a radiolabelled ligand to the **RUP3** receptor. Absent the use of known ligands for the **RUP3** receptor and radiolabels thereof, compounds of Formula **(I)** can be labelled with a radioisotope and used in an assay for evaluating the affinity of a test compound to the **RUP3** receptor.

A radiolabelled **RUP3** compound of Formula **(I)** can be used in a screening assay to identify/evaluate compounds. In general terms, a newly synthesized or identified compound (i.e., test compound) can be evaluated for its ability to reduce binding of the "radiolabelled compound of Formula (**I**)" to the **RUP3** receptor. Accordingly, the ability to compete with the "radio-labelled compound of Formula (**I**)" or **Radiolabelled RUP3 Ligand** for the binding to the **RUP3** receptor directly correlates to its binding affinity of the test compound to the **RUP3** receptor.

### ASSAY PROTOCOL FOR DETERMINING RECEPTOR BINDING FOR RUP3:

### A. RUP3 RECEPTOR PREPARATION

293 cells (human kidney, ATCC), transiently transfected with 10 ug human **RUP3** receptor and 60 ul Lipofectamine (per 15-cm dish), were grown in the dish for 24 hours (75% confluency) with a media change and removed with 10 ml/dish of Hepes-EDTA buffer ( 20mM Hepes + 10 mM EDTA, pH 7.4). The cells were then centrifuged in a Beckman Coulter centrifuge for 20 minutes, 17,000 rpm (JA-25.50 rotor). Subsequently, the pellet was resuspended in 20 mM Hepes + 1 mM EDTA, pH 7.4 and homogenized with a 50- ml Dounce homogenizer and again centrifuged. After removing the supernatant, the pellets were stored at -80°C, until used in binding assay. When used in the assay, membranes were thawed on ice for 20 minutes and then 10 mL of incubation buffer (20 mM Hepes, 1 mM $MgCl_2$, 100 mM NaCl, pH 7.4) added. The membranes were then vortexed to resuspend the crude membrane pellet and homogenized with a Brinkmann PT-3100 Polytron homogenizer for 15 seconds at setting 6. The concentration of membrane protein was determined using the BRL Bradford protein assay.

### B. BINDING ASSAY

For total binding, a total volume of 50ul of appropriately diluted membranes (diluted in assay buffer containing 50mM Tris HCl (pH 7.4), 10mM $MgCl_2$, and 1mM EDTA; 5-50ug protein) is added to 96-well polyproylene microtiter plates followed by addition of 100ul of assay buffer and 50ul of **Radiolabelled RUP3 Ligand**. For nonspecific binding, 50 ul of assay buffer is added instead of 100ul and an additional 50ul of 10uM cold **RUP3** is added before 50ul of **Radiolabelled RUP3 Ligand** is added. Plates are then incubated at room temperature for 60-120 minutes. The binding reaction is terminated by filtering assay plates through a Microplate Devices GF/C Unifilter filtration plate with a Brandell 96-well plate harvestor followed by washing with cold 50 mM Tris HCl, pH 7.4 containing 0.9% NaCl. Then, the bottom of the filtration plate are sealed, 50ul of Optiphase Supermix is added to each well, the top of the plates are sealed, and plates are counted in a Trilux MicroBeta scintillation counter. For compound competition studies, instead of adding 100ul of assay buffer, 100ul of appropriately diluted test compound is added to appropriate wells followed by addition of 50 ul of **Radiolabelled RUP3 Ligand.**

## C. CALCULATIONS

The test compounds are initially assayed at 1 and 0.1 μM and then at a range of concentrations chosen such that the middle dose would cause about 50% inhibition of a **Radio-RUP3 Ligand** binding (i.e., $IC_{50}$). Specific binding in the absence of test compound ($B_O$) is the difference of total binding ($B_T$) minus non-specific binding (NSB) and similarly specific binding (in the presence of test compound) (B) is the difference of displacement binding ($B_D$) minus non-specific binding (NSB). $IC_{50}$ is determined from an inhibition response curve, logit-log plot of % $B/B_O$ vs concentration of test compound.

$K_i$ is calculated by the Cheng and Prustoff transformation:

$$K_i = IC_{50} / (1+[L] / K_D)$$

where [L] is the concentration of a Radio-labeled **RUP3** Ligand used in the assay and $K_D$ is the dissociation constant of a Radio-labeled **RUP3** Ligand determined independently under the same binding conditions.

### Example 3:

The compounds of the invention and their synthesis are further illustrated by the following examples. The following examples are provided to further define the invention without, however, limiting the invention to the particulas of these examples. The compounds described herein are named according to the CS Chem Draw Ultra Version 7.0.1. In certain instances common names are used and it is understood that these common names would be recognized by those skilled in the art.

**Chemistry**: Proton nuclear magnetic resonance ($^1H$ NMR) spectra were recorded on a Varian Mercury Vx-400 equipped with a 4 nucleus auto switchable probe and z-gradient or a Bruker Avance-400 equipped with a QNP (Quad Nucleus Probe) or a BBI (Broad Band Inverse) and z-gradient. Chemical shifts are given in parts per million (ppm) with the residual solvent signal used as reference. NMR abbreviations are used as follows: s = singlet, d = doublet, t = triplet, q = quartet, m = multiplet, br = broad. Microwave irradiations were carried out using the Emyrs Synthesizer (Personal Chemistry). Thin-layer chromatography (TLC) was performed on silica gel 60 $F_{254}$ (Merck), preparatory thin-layer chromatography (prep TLC) was preformed on PK6F silica gel 60 A 1 mm plates (Whatman), and column chromatography was carried out on a silica gel column using Kieselgel 60, 0.063-0.200 mm (Merck). Evaporation was done in vacuo on a Buchi rotary evaporator. Celite 545 ® was used during palladium filtrations.

LCMS specs: 1) PC: HPLC-pumps: LC-10AD *VP*, Shimadzu Inc.; HPLC system controller: SCL-10A *VP*, Shimadzu Inc; UV-Detector: SPD-10A *VP*, Shimadzu Inc; Autosampler: CTC HTS, PAL, Leap Scientific; Mass spectrometer: API 150EX with Turbo Ion Spray source, AB/MDS Sciex; Software: Analyst 1.2. 2) Mac: HPLC-pumps: LC-8A *VP*, Shimadzu Inc; HPLC system controller: SCL-10A *VP*, Shimadzu Inc. UV-Detector: SPD-10A *VP*, Shimadzu Inc; Autosampler: 215 Liquid Handler, Gilson Inc; Mass spectrometer: API 150EX with Turbo Ion Spray source, AB/MDS Sciex Software: Masschrom 1.5.2.

### Example 3.1: Preparation of 5-Methoxypyrimidine-4,6-diol.

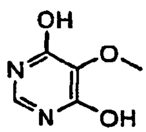

A 2-liter, three-necked, round-bottomed flask equipped with a mechanical stirrer and a reflux condenser was dried at 100 °C and cooled to 25 °C under $N_2$. Methanol (260 mL) was added, followed by a solution of sodium methoxide in methanol (25% by weight, 670 mL, 3.10 mol). The resulting solution was cooled to 0 °C. Dimethyl methoxymalonate (151 g, 0.930 mol) was added in one portion, followed by formamidine acetate (100 g, 0.96 mol). The temperature was kept below 10 °C. The resulting suspension was then stirred at 0 °C for 30 min, and refluxed for 1 h. The resulting suspension was cooled to 0 °C and quenched with concentrated HCl (250 mL) over 30 min, during which time the temperature was kept below 10 °C. Stirring was continued at 5 °C for 30 min. The suspension was then filtered. The filter cake was re-suspended in water (1000 mL) and refluxed until a nearly clear solution was obtained. The solution

was filtered while hot. The filtrate was cooled to 0-5 °C and stirred for 1 h. The solid precipitate was collected by filtration, washed with cold methanol (500 mL), and dried in a vacuum oven at 50 °C for 1 h to give the title compound (96.2 g, 73% yield) as a white solid. Exact Mass calculated for $C_5H_6N_2O_3$: 142.0, LCMS *m/z* = 143.2 ($M+H^+$); $^1H$ NMR (400 MHz, DMSO-$d_6$) δ 3.63 (s, 3 H), 7.81 (s, I H), 11.75 (br s, 2 H).

**Example 3.2: Preparation of 4,6-Dichloro-5-methoxypyrimidine.**

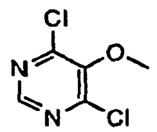

A suspension of 5-methoxypyrimidine-4,6-diol (96.0 g, 676 mmol) and triethylamine (95.0 mL, 680 mmol) in anhydrous toluene (1.2 L) was heated to 100-105 °C, and a solution of $POCl_3$ (140 mL, 1.5 mol) in anhydrous toluene (200 mL) was added over 30 min. The mixture was refluxed for 1 h and cooled to ambient temperature. The toluene layer was decanted and ice was added. The dark, heavier layer was separated, more ice was added, and the mixture was extracted with toluene (2 X 200 mL). The toluene extracts were combined, and the aqueous layer was discarded. The organic extract was then washed with saturated $NaHCO_3$ (2 X 300 mL), brine (400 mL), dried over $MgSO_4$, and concentrated to give the title compound (103.4 g, 86% yield) as a white solid. $^1H$ NMR ($CDCl_3$) δ 4.00 (s, 3 H), 8.55 (s, 1 H).

**Example 3.3: Preparation of 4-Hydroxy-piperidine-1-carboxylic Acid Isopropyl Ester.**

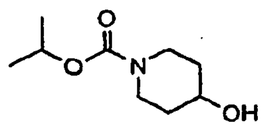

A magnetically stirred solution of 4-hydroxypiperidine (70.3 g, 695 mmol) and *N*,*N*-diisopropylethylamine (105 mL, 600 mmol) in dichloromethane (1.0 L) was cooled to 10 °C under $N_2$. A solution of isopropyl chloroformate (1.0 M in toluene, 580 mL, 580 mmol) was added dropwise over 2 h, maintaining a temperature of 10-15 °C. The reaction mixture was stirred for an additional 2 h and then extracted with 1 N HCl (1.2 L). The organic extract was dried over $MgSO_4$, and the solvent was removed under reduced pressure to give the title compound (90.3 g, 83% yield) as a pale straw-colored oil. Exact Mass calculated for $C_9H_{17}NO_3$: 187.1, Found: LCMS *m/z* = 188.2 ($M+H^+$), 210.3 ($M+Na^+$); $^1H$ NMR (400 MHz, $CDCl_3$) δ 1.24 (d, *J* = 6.3 Hz, 6 H), 1.47 (m, 2 H), 1.86 (m, 2 H), 3.08 (m, 2 H), 3.86 (m, 3 H), 4.90 (m, 1 H).

**Example 3.4: Preparation of 4-(6-Chloro-5-methoxy-pyrimidin-4-yloxy)-piperidine-1-carboxylic Acid Isopropyl Ester.**

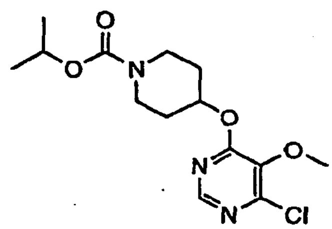

A solution of 4-hydroxy-piperidine-1-carboxylic acid isopropyl ester (71.0 g, 380 mmol) and 4,6-dichloro-5-methoxypyrimidine (71.6 g, 400 mmol) in anhydrous THF (1 L) was cooled to 5 °C under $N_2$. A solution of potassium *t*-butoxide (1.0 M in THF, 380 mL, 380 mmol) was added dropwise over 1 h. The reaction temperature was kept under

10 °C during addition. The reaction mixture was stirred at 5-10 °C for 1 h, quenched with saturated $NH_4Cl$ (200 mL), and diluted with ether (1 L) and water (1 L). The aqueous phase was separated and discarded. The organic extract was washed with brine (800 mL), dried over $MgSO_4$, and then concentrated. The residue was dissolved in hexane (400 mL) and filtered over Celite™ to remove a small amount of brown solid. The solvent was removed from the filtrate to afford a pale amber oil which gradually crystallized to give the title compound (130 g, 98.6% yield) as a pale amber solid. Exact Mass calculated for $C_{14}H_2OClN_3O_4$: 329.1, Found: LCMS *m/z* = 330.2 (M+H+), $^1H$ NMR (400 MHz, $CDCl_3$) δ 1.25 (d, *J* = 6.2 Hz, 6 H), 1.82 (m, 2 H), 2.02 (m, 2 H), 3.40 (m, 2 H), 3.80 (m, 2 H), 3.91 (s, 3 H), 4.95 (m, 1 H), 5.39 (m, I H), 8.27 (s, 1 H).

**Example 3.5: Preparation of 4-[6-(6-Bromo-2-methyl-pyridin-3-ylamino)-5-methoxy-pyrimidin-4-yloxy]-piperidine-1-carboxylic Acid Isopropyl Ester.**

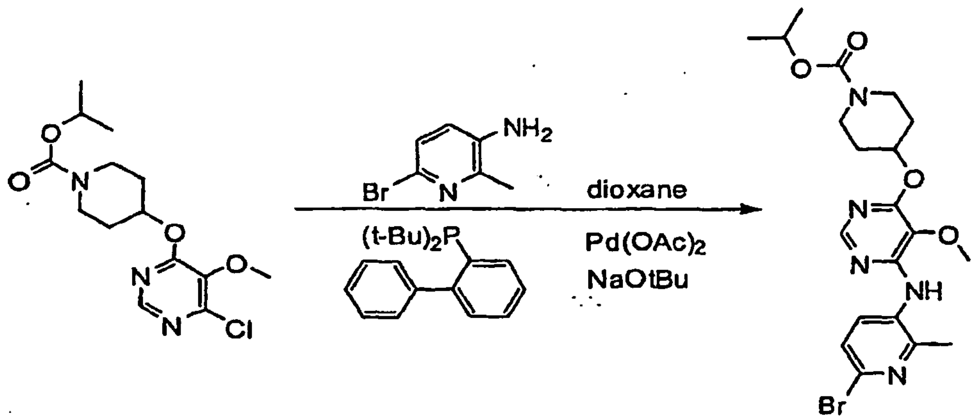


A mixture of 4-(6-chloro-5-methoxy-pyrimidin-4-yloxy)-piperidine-1-carboxylic acid isopropyl ester (712 mg, 2.20 mmol), 6-bromo-2-methylpyridin-3-amine (445 mg, 2.40 mmol), palladium acetate (25 mg, 0.11 mmol), 2-(di-t-butylphosphino) biphenyl (65 mg, 0.22 mmol) and sodium *tert*-butoxide (315 mg, 3.30 mmol) in dioxane (15 mL) was heated under microwave irradiation at 120 °C for 2 h. The mixture was purified by HPLC to give the title compound (TFA salt, 104 mg, 8% yield) as an oil. Exact mass calculated for $C_{20}H_{26}BrN_5O_4$: 479.1, Found: LCMS *m/z* = 480.0 (M+H+); $^1H$ NMR (400 MHz, $CDCl_3$) δ 1.28 (d, 6 H), 1.84-1.87 (m, 2 H), 2.02-2.08 (m, 2 H), 2.58 (s, 3 H), 3.40-3.47 (m, 2 H), 3.73 (s, 3 H), 3.77-3.82 (m, 2 H), 4.93-4.97 (m, 1 H), 5.41-5.43 (m, 1 H), 7.44-7.46 (m, 1 H), 7.91-7.93 (m, 1 H), 8.24 (s, 1 H), 8.70 (s br, 1 H).

**Example 3.6: Preparation of 4-[5-Methoxy-6-(2-methyl-6-[1,2,4]triazol-1-yl-pyridin-3-ylamino)-pyrimidin4-yloxy]-piperidine-1-carboxylic Acid Isopropyl Ester (Method 1).**

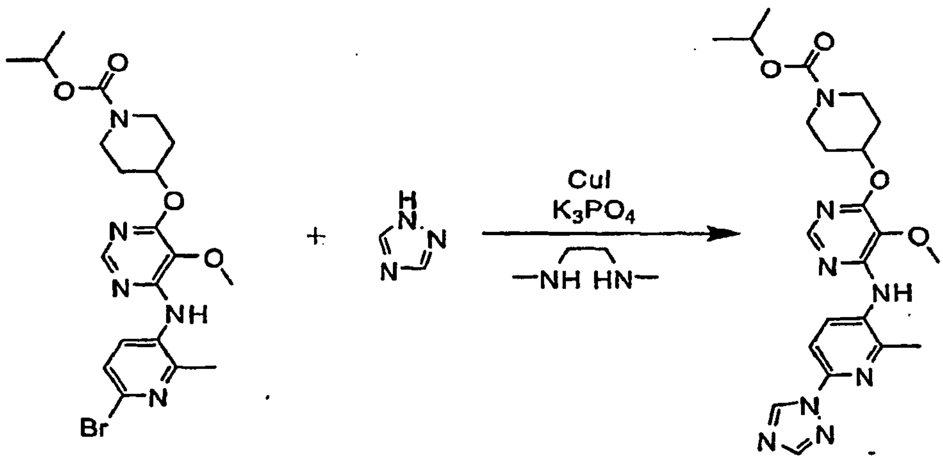


A mixture of 4-[6-(6-bromo-2-methyl-pyridin-3-ylamino)-5-methoxy-pyrimidin-4-yloxy]-piperidine-1-carboxylic acid isopropyl ester (496 mg, 1.03 mmol), 1H-1,2,4-triazole (59 mg, 0.86 mmol), copper(I) iodide (8.2 mg, 0.040 mmol), potassium phosphate (384 mg, 1.80 mmol), and $N^1$,$N^2$-dimethylethane-1,2-diamine (0.02 ml, 0.17 mmol) in DMF (4 mL) was heated at 150 °C for 2 h. The mixture was purified by HPLC. The collected fractions were combined and extracted with dichloromethane/1 M NaOH. The organic layer was dried over $MgSO_4$, filtered, and concentrated. The residue was recrystallized from ethanol to give the title compound (58 mg, 14%) as a solid. Exact mass calculated for $C_{22}H_{28}N_8O_4$: 468.2, Found: LCMS *m/z* = 469.5 (M+H$^+$); $^1$H NMR (400 MHz, $CDCl_3$) δ 1.26 (d, *J* = 6.32 Hz, 6 H); 1.82-1.85 (m, 2 H), 2.02-2.06 (m, 2 H), 2.59 (s, 3 H), 3.38-3.45 (m, 2 H), 3.79-3.80 (m, 2 H), 3.99 (s, 3 H), 4.95 (sept, *J* = 6.32 Hz, 1 H), 5.37-5.39 (m, 1 H), 7.01 (s, 1 H), 7.76 (d, *J* = 8.34 Hz, 1 H), 8.08 (s, 1 H), 8.14 (s, 1 H), 8.63 (d, *J* = 8.34 Hz, 1 H), 9.12 (s, 1 H).

**Example 3.7: Preparation of 2-Methyl-6-[1,2,4]triazol-1-yl-pyridin-3-ylamine (Method 1).**

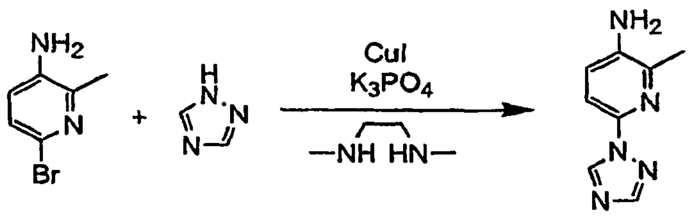


A mixture of 6-bromo-2-methylpyridin-3-amine (20.4 g, 109 mmol), 1H-1,2,4-triazole (6.28 g, 91.0 mmol), copper (I) iodide (866 mg, 4.55 mmol), potassium phosphate (40.5 g, 191 mmol), and $N^1$,$N^2$-dimethylethane-1,2-diamine (1.96 ml, 18.2 mmol) in DMF (200 mL) was heated at 110 °C for 66 h. The mixture was extracted with ethyl acetate/$H_2O$ six times. The organic phase was dried over $MgSO_4$, filtered and concentrated. The residue was purified by column chromatography on silica gel with hexane/ethyl acetate (1:1 to 0:1, v/v). The collected fractions were concentrated and further purified by recrystallization from ethanol to give the title compound (8.72 g, 54%) as a brown solid. Exact mass calculated for $C_8H_9N_5$: 175.1, Found: LCMS *m/z* = 176.3 (M+H$^+$); $^1$H NMR (400 MHz, $CDCl_3$) δ 2.44 (s, 3 H), 3.75 (s, 2 H), 7.09 (d, *J* = 8.34 Hz, 1 H), 7.54 (d, *J* = 8.34 Hz, 1 H), 8.04 (s, 1H), 9.02 (s, 1 H).

**Example 3.8: Preparation of 2-Methyl-6-[1,2,4]triazol-1-yl-pyridin-3-ylamine (Method 2).**

**Step A: Preparation of 2-Methyl-3-nitro-6-(1H-1,2,4-triazol-1-yl)pyridine.**

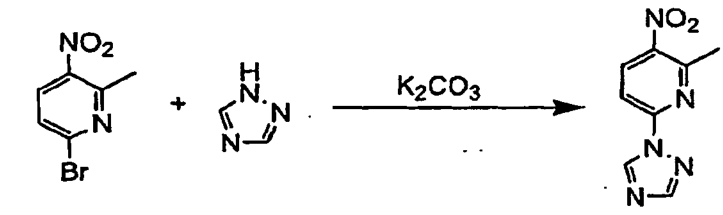


A mixture of 6-bromo-2-methyl-3-nitropyridine (5.0 g, 23 mmol), 1H-1,2,4-triazole (1.6 g, 23 mmol), and potassium carbonate (3.2 g, 23 mmol) in DMSO (10 mL) was stirred at room temperature for 17 h. The reaction mixture was poured into ice-water (1 L) and stirred until all the ice had melted. The ice-cold solution was filtered to give the title compound (3.4 g, 72%) as a dark purple solid. Exact mass calculated for $C_8H_7N_5O_2$: 205.1, Found: LCMS *m/z* = 206.2 ($M+H^+$).

**Step B: Preparation of 2-Methyl-6-[1,2,4]triazol-1-yl-pyridin-3-ylamine.**

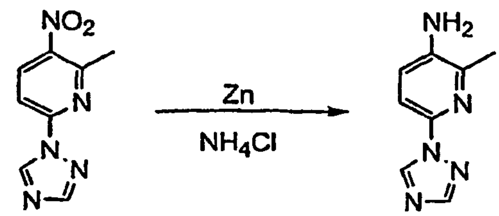


To a suspension of zinc dust (6.80 g, 104 mmol) and 2 M aqueous ammonium chloride solution (52 mL), 2-methyl-3-nitro-6-(1H-1,2,4-triazol-1-yl)pyridine (4.30 g, 20.8 mmol) in ethyl acetate (70 mL) was added dropwise at 0 °C *via* an addition funnel. The mixture was stirred at room temperature for 17 h and filtered through Celite™. The filtrate was then extracted with ethyl acetate. The organic phase was separated, dried over $MgSO_4$, filtered, and concentrated. The residue was recrystallized from ethanol to give the title compound as a brown solid (yield). Exact mass calculated for $C_8H_9N_5$: 175.1, Found: LCMS *m/z* = 176.3 ($M+H^+$); $^1H$ NMR (400 MHz, DMSO-$d_6$) δ 2.33 (s, 3 H), 5.39 (s, 2H), 7.14 (d, 1 H), 7.41 (d, 1 H), 8.16 (s, 1 H), 9.06 (s, 1 H).

**Example 3.9: Preparation of 4-[5-Methoxy-6-(2-methyl-6-[1,2,4]triazol-1-yl-pyridin-3-ylamino)-pyrimidin-4-yloxy]-piperidine-1-carboxylic acid isopropyl ester (Method 2).**

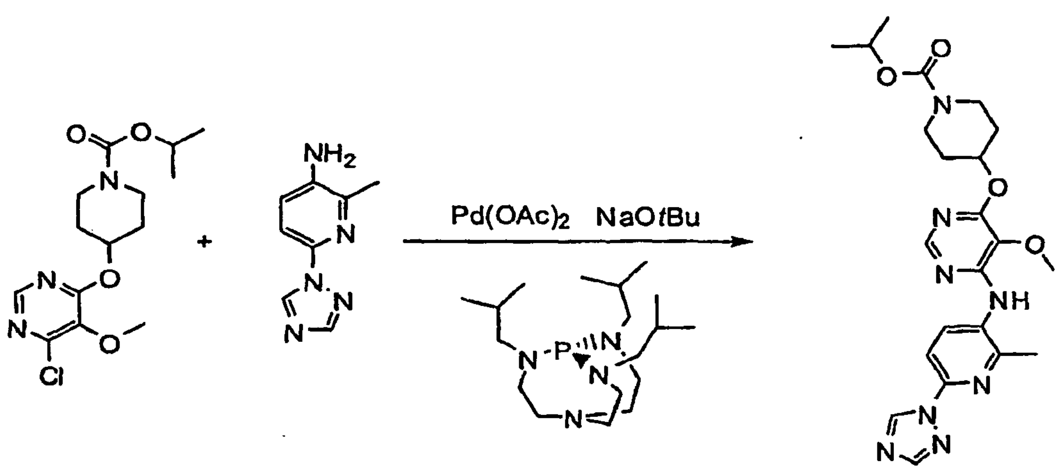

A mixture of 4-(6-chloro-5-methoxy-pyrimidin-4-yloxy)-piperidine-1-carboxylic acid isopropyl ester (13.7 g, 41.5 mmol), 2-methyl-6-[1,2,4]triazol-1-yl-pyndin-3-ylamine (8.10 g, 46.2 mmol), palladium acetate (97.7 mg, 0.435 mmol), 2,8,9-triisobutyl-2,5,8,9-tetraaza-1-phosphabicyclo[3.3.3]undecane (309 μl, 0.870 mmol) and sodium tert-butoxide (10.0 g, 104 mmol) in dioxane (200 ml) was heated at 90 °C for 2 h. The crude mixture was extracted with ethyl acetate/$H_2O$ and washed with brine. The organic layer was dried over $MgSO_4$, filtered, and concentrated. The residue was purified by column chromatography on silica gel with hexane/ethyl acetate (1:1, v/v). The collected fractions were concentrated and further purified by recrystallization from ethanol to give the title compound (9.60 g, 46%) as a white solid. Exact mass calculated for $C_{22}H_{28}N_8O_4$: 468.2, Found: LCMS *m/z* = 469.5 ($M+H^+$); $^1$H NMR (400 MHz, $CDCl_3$) δ 1.26 (d, *J* = 6.32 Hz, 6 H), 1.82-1.85 (m, 2 H), 2.02-2.06 (m, 2 H), 2.59 (s, 3 H), 3.38-3.45 (m, 2 H), 3.79-3.80 (m, 2 H), 3.99 (s, 3 H), 4.95 (sept, *J* = 6.32 Hz, 1 H), 5.37-5.39 (m, 1 H), 7.01 (s, 1 H), 7.76 (d, *J* = 8.34 Hz, 1 H), 8.08 (s, 1 H), 8.14 (s, 1 H), 8.63 (d, *J* = 8.34 Hz, 1 H), 9.12 (s, 1 H).

**Example 4: Protocol for RUP3 Dose Responses in Melanophores**

Melanophores are maintained in culture as reported by Potenza, M. N. and Lerner, M. R., in Pigment Cell Research, Vol. 5, 372-378, 1992 and transfected with the RUP3 expression vector (pCMV) using electroporation. Following electroporation, the transfected cells are plated into 96 well plates for the assay. The cells are then allowed to grow for 48 hours in order to both recover from the electroporation procedure and attain maximal receptor expression levels.

On the assay day, the growth medium on the cells is replaced with serum-free buffer containing 10nM melatonin. The melatonin acts via an endogenous Gi-coupled GPCR in the melanophores to lower intracellular cAMP levels. In response to lowered cAMP levels, the melanophores translocate their pigment to the center of the cell. The net effect of this is a significant decrease in the absorbance reading of the cell monolayer in the well, measured at 600-650nM.

After a 1-hour incubation in melatonin, the cells become completely pigment-aggregated. At this point a baseline absorbance reading is collected. Serial dilutions of test compounds are then added to the plate and compounds that stimulate RUP3 produce increases in intracellular cAMP levels. In response to these increased cAMP levels, the melanophores translocate their pigment back into the cell periphery. After one hour, stimulated cells are fully pigment-dispersed. The cell monolayer in the dispersed state absorbs much more light in the 600-650nm range. The measured increase in absorbance compared to the baseline reading allows one to quantitate the degree of receptor stimulation and plot a dose-response curve.

The compound 4-[5-Methoxy-6-(2-methyl-6-[1,2,4]triazol-1-yl-pyridin-3-ylamino)-pyrimidin-4-yloxy]-piperidine-1-carboxylic acid isopropyl ester, as shown in Formula (I), is a potent agonist of the RUP3 receptor in a number of different species, $EC_{50}$ = 2 nM (human), 4 nM (dog), 57 nM (mouse), and 81 nM (rat).

**Example 5: Rat Dose-range PK Study for 4-[5-Methoxy-6-(2-methyl-6-[1,2,4]triazol-1-yl-pyridin-3-ylamino)-pyrimidin-4-yloxy]-piperidine-1-carboxylic acid isopropyl ester. Animal, compound formulation, dosing, and blood sample collection:**

Male SD rats (250-300 g) were purchased from Charles River Laboratory; upon reciept, animals were placed under light-dark cycle (6:30 am - 6:30 pm lights on). They were allowed *ad libitum* access to water and 4 pieces of food daily (Purina Meals Rodent Diet, Product Number 5001).

Compound formulations were prepared as following: The IV injection formulation was prepared in 20% hydroxypropyl-beta-cyclodextrin with concentration of 0.667 mg/mL. The PO formulations were prepared in 0.5% hydroxypropyl methylcellulose with concentrations of 0.3, 3, and 30 mg/Kg. The dosing volume for IV injection was 3 mL/Kg and for PO adiministration was 10 mL/Kg. Four rats were used for each dose group. The dose of IV injection was 2 mg/kg and the dose of PO was 3, 30, or 300 mg/Kg, respectively.

All rats (4 rats per group, housed individualy) were fasted overnight prior to in-life phase. On the next morning, rats were received an IV (via tail vein injection) injection or gavage dose of compound starting at 8 am (IV) and 9 am (PO). Next, each rat were orbital bled at 0.085, 0.25, 0.5, 1, 2, 4, 6, 8, and 24 hr (IV) or 0.5, 1, 2, 4, 6, 8, and 24 hr (PO) to collect blood samples for PK analysis.

The blood samples were collected via orbital bleeding into tubes containing EDTA, 0.25 mL blood each time. These samples were put on ice and within 2 hours plasma was prepared by centrifugation at 3,000 rpm for 30 min at 4°C. 100 μl of plasma were transferred into a 96-tube box for PK analysis.

**Sample analysis:**

Plasma samples were prepared as follows. Two hundred microliters of acetonitrile containing internal standard was added to 100 μL of plasma to precipitate proteins. Samples were centrifuged at 3000 g for 5 minutes and supernatant

removed for analysis by LC-MS-MS. Calibration standards and quality control samples were prepared by adding a known volume of standard stock solution (50% methanol, 50% $H_2O$) directly into blank plasma and treated identically to collected plasma samples. Calibration standards were typically prepared in the range of 2.0 ng/mL to 10 μg/mL with linear regression for quantitation. These sample preparation steps were automated using a liquid handling workstation (Tomtec Quadra 96) in the 96-well format. Reversed phase LC-MS-MS analysis was performed using either multiple reaction or selected ion monitoring for detection of characteristic ions for each drug candidate and the internal standard used was propranolol for positive ions or chloramphenicol for negative ions.

**Data Interpretation:**

Results were calculated by noncompartmental analysis using WinNonlin Pro version 3.1 based on plasma concentration - time profiles for individual animals. Plasma levels were determined as described above and the oral and intravenous area under the concentration vs. time curve (AUC was calculated using the linear trapezoidal rule up to the last measurable concentration and was then extrapolated to infinity) were compared to determine the % bioavailability (%F) by the following formula: Dose (IV)*AUC (oral) / Dose (oral)*AUC (IV).

There may be significant variation within the individual animals and the analytical method and that variation was evidenced by the %CV. AUMC was the first statistical moment of the AUC and was used to calculate the mean residence time (MRT= AUMC/AUC), which was the average time the compound was in the animal. The $C_{max}$ represented the maximum concentration observed, the $T_{max}$ was the time to reach that maximum concentration and the $T_{1/2}$ was the calculated terminal half-life of the compound in plasma using the slope of a log concentration vs time plot if there were sufficient elimination phase data points (at least three data points in the terminal phase excluding the Cmax). Systemic clearance (CL=Dose(IV)/AUC(IV)) was the volume of fluid (containing compound) from which compound was removed completely per unit time. Volume of distribution at steady state (Vss=CL*MRT) was the extent of distribution of a drug from the plasma to the tissues at steady state.

The RUP3 agonist, 4-[5-Methoxy-6-(2-methyl-6-[1,2,4]triazol-1-yl-pyridin-3-ylamino)-pyrimidin-4-yloxy]-piperidine-1-carboxylic acid isopropyl ester, showed essentially linear dose escalation pharmacokinetics, see Figure 1.

The numerical data associated with each of the compounds shown in Figure 1 can be found in the table below.

**Dose Escalation Pharmacokinetics AUC vs. Dose**

| | AUC24hr (hr•μg/mL) | | | | | |
|---|---|---|---|---|---|---|
| **Compound** | **3 mg** | **10 mg** | **30 mg** | **100 mg** | **300 mg** | **1000 mg** |
| A | - | 2.59 | 9.93 | 133.37 | 96.97 | 377.45 |
| B | 2.12 | - | 5.99 | - | 11.03 | - |
| C | 14.91 | - | 65.91 | - | 418.53 | - |
| D | 5.6 | - | 29.38 | - | 54.69 | - |
| E | 4.73 | - | 55.9 | - | 515.32 | - |
| F | 0.51 | - | 6.77 | - | 12.19 | - |
| G | 1.66 | - | 6.47 | - | 33.85 | - |
| Cmpd of Formula **(I)** | 3.59 | - | 79.82 | - | 285.99 | - |

Also shown in Figure 1 is Compound A [i.e., 4-[1-(2-Fluoro-4-methanesulfonyl-phenyl)-1H-pyrazolo[3,4-d]pyrimidin-4-yloxy]-piperidine-1-carboxylic acid isopropyl ester] that is described in the genus found in PCT/US2004/022417; Compound B [i.e., (2-Fluoro-4-methanesulfonyl-phenyl)-{6-[1-(3-isopropyl-[1,2,4]oxadiazol-5-yl)-piperidin-4-yloxy]-5-methyl-pyrimidin-4-yl}-amine] that is described in the genus found in PCT/US2004/022327; Compound C [i.e., 4-[6-(6-Methanesulfonyl-2-methyl-pyfidin-3-ylamino)-5-methoxy-pyrimidin-4-yloxy]-piperidine-1-carboxylic acid isopropyl ester] that is described in the genus found in PCTUS/2006/000567; Compound D [i.e., 4-[6-(6-Methanesulfonyl-4-methyl-pyridin-3-ylamino)-5-methoxy-pyrimidin-4-yloxy]-piperidine-1-carboxylic acid isopropyl ester] that is described in the genus found in PCTUS/2006/000567; Compound E [i.e., 4-[6-(6-methanesulfonyl-2-methyl-pyridin-3-ylamino)-5-methyl-pyrimidin-4-yloxy]-piperidine-1-carboxylic acid isopropyl ester] that is described in the genus found in PCT/US20041022327; Compound F that is described in the genus found in PCT/US2004/001267; and Compound G [i.e., {6-[1-(3-Isopropyl-[1,2,4]oxadiazol-5-yl)-piperidin-4-yloxy]-5-methoxy-pyrimidin-4-yl}-(6-methanesulfonyl-2-methyl-pyridin-3-yl)-amine] that is described in the genus found in PCTUS/20061000567.

While RUP3 agonists can be useful as therapeutics in the treatment of a number of metabolic-related disorders

as described herein, compounds that exhibit linear dose escalation pharmacolinetic properties, such as 4-[5-Methoxy-6-(2-methyl-6-[1,2,4]triazol-1-yl-pyridin-3-ylamino)-pyrimdin-4-yloxy]-piperidine-1-carboxylic acid isopropyl ester, are particularly beneficial for a variety of reasons. For example, compounds with linear exposure vs. dose relationship have the following benefits:

The pharmacokinetic parameters are more predictable when different doses are administered or when the drug is given through different routes of administration or as single or multiple doses. Patients are less likely to be overdosed when doses are slightly increased.

These compounds have better absorption and may have enhanced oral bioavailability. Drug with nonlinearity may have decreased oral bioavailability due to several possible reasons including drug concentration approaching the drug's solubility limit in the GI tract, or a saturable transport system for absorption.

During preclinical drug development, these compounds will be able to achieve high exposure when dosed at higher doses.

## Claims

1. A compound selected from compounds of Formula (I) and pharmaceutically acceptable salts, solvates, and hydrates thereof:

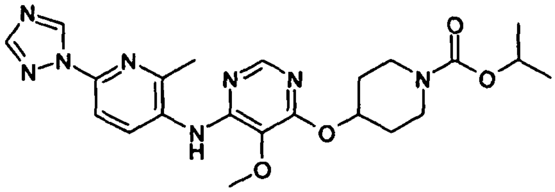

(I)

2. A pharmaceutical composition comprising a compound according to claim 1 and a pharmaceutically acceptable carrier.

3. A method of producing a pharmaceutical composition comprising admixing a compound according to claim 1 and a pharmaceutically acceptable carrier.

4. A compound according to claim 1 for use in a method of treatment of the human or animal body by therapy.

5. A compound according to claim 1 for use in a method of treatment of a metabolic-related disorder.

6. A compound according to claim 1 for use in a method of treatment of type I diabetes, type II diabetes, inadequate glucose tolerance, insulin resistance, hyperglycemia, hyperlipidemia, hypertriglyceridemia, hypercholesterolemia, dyslipidemia, or syndrome X.

7. A compound according to claim 1 for use in a method of treatment of type II diabetes.

8. A compound according to claim 1 for use in a method of treatment of dyslipidemia.

9. A compound according to claim 1 for use in a method of treatment of obesity.

10. A compound according to claim 1 for use in a method of:

    decreasing food intake of an individual or
    inducing satiety in an individual; or
    controlling or decreasing weight gain of an individual.

11. Use of a compound according to claim 1 for production of a medicament for treatment of a metabolic-related disorder.

12. Use of a compound according to claim 1 for production of a medicament for treatment of type I diabetes, type II

diabetes, inadequate glucose tolerance, insulin resistance, hyperglycemia, hyperlipidemia, hypertriglyceridemia, hypercholesterolemia, dyslipidemia, or syndrome X.

**13.** Use of a compound according to claim 1 for production of a medicament for treatment of type II diabetes.

**14.** Use of a compound according to claim 1 for production of a medicament for treatment of dyslipidemia.

**15.** Use of a compound according to claim 1 for production of a medicament for treatment of obesity.

**16.** Use of a compound according to claim 1 for production of a medicament for:

decreasing food intake in an individual or
inducing satiety in an individual; or
controlling or decreasing weight gain in an individual.

**Patentansprüche**

**1.** Verbindung, die aus Verbindungen der Formel (I) und pharmazeutisch annehmbaren Salzen, Solvaten und Hydraten davon ausgewählt ist:

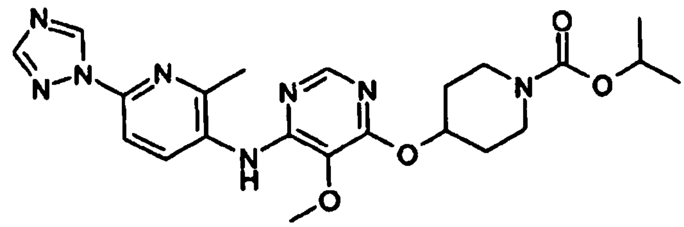

(I)

**2.** Pharmazeutische Zusammensetzung, die eine Verbindung nach Anspruch 1 und einen pharmazeutisch annehmbaren Träger umfasst.

**3.** Verfahren zur Herstellung einer pharmazeutischen Zusammensetzung, welches das Vermischen einer Verbindung nach Anspruch 1 und eines pharmazeutisch annehmbaren Trägers umfasst.

**4.** Verbindung nach Anspruch 1 zur Verwendung in einem Verfahren zur Behandlung des Körpers eines Menschen oder Tiers durch Therapie.

**5.** Verbindung nach Anspruch 1 zur Verwendung in einem Verfahren zur Behandlung einer Stoffwechselstörung.

**6.** Verbindung nach Anspruch 1 zur Verwendung in einem Verfahren zur Behandlung von Typ-I-Diabetes, Typ-II-Diabetes, Glucosetoleranzstörungen, Insulinresistenz, Hyperglykämie, Hyperlipidämie, Hypertriglyceridämie, Hypercholesterinämie, Dyslipidämie oder Syndrom X.

**7.** Verbindung nach Anspruch 1 zur Verwendung in einem Verfahren zur Behandlung von Typ-II-Diabetes.

**8.** Verbindung nach Anspruch 1 zur Verwendung in einem Verfahren zur Behandlung von Dyslipidämie.

**9.** Verbindung nach Anspruch 1 zur Verwendung in einem Verfahren zur Behandlung von Obesität.

**10.** Verbindung nach Anspruch 1 zur Verwendung in einem Verfahren zur:

Verringerung der Nahrungsaufnahme eines Individuums; oder
Erzeugung eines Sättigungsgefühls in einem Individuum; oder

Kontrolle oder Senkung der Gewichtszunahme eines Individuums.

**11.** Verwendung einer Verbindung nach Anspruch 1 zur Herstellung eines Medikaments zur Behandlung einer Stoffwechselstörung.

**12.** Verwendung einer Verbindung nach Anspruch 1 zur Herstellung eines Medikaments zur Behandlung von Typ-I-Diabetes, Typ-II-Diabetes, Glucosetoleranzstörungen, Insulinresistenz, Hyperglykämie, Hyperlipidämie, Hypertriglyceridämie, Hypercholesterinämie, Dyslipidämie oder Syndrom X.

**13.** Verwendung einer Verbindung nach Anspruch 1 zur Herstellung eines Medikaments zur Behandlung von Typ-II-Diabetes.

**14.** Verwendung einer Verbindung nach Anspruch 1 zur Herstellung eines Medikaments zur Behandlung von Dyslipidämie.

**15.** Verwendung einer Verbindung nach Anspruch 1 zur Herstellung eines Medikaments zur Behandlung von Obesität.

**16.** Verwendung einer Verbindung nach Anspruch 1 zur Herstellung eines Medikaments zur:

Verringerung der Nahrungsaufnahme eines Individuums; oder
Erzeugung eines Sättigungsgefühls in einem Individuum; oder
Kontrolle oder Senkung der Gewichtszunahme eines Individuums.

## Revendications

**1.** Composé sélectionné parmi des composés de la Formule (I) et des sels, solvates et hydrates pharmaceutiquement acceptables de celui-ci:

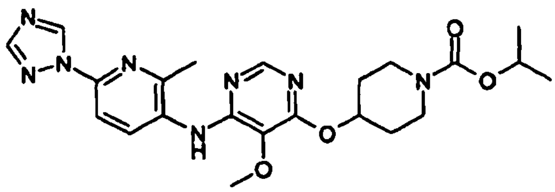

**(I)**

.

**2.** Composition pharmaceutique comprenant un composé selon la revendication 1 et un support pharmaceutiquement acceptable.

**3.** Procédé de production d'une composition pharmaceutique comprenant le mélange d'un composé selon la revendication 1 et d'un support pharmaceutiquement acceptable.

**4.** Composé selon la revendication 1 pour utilisation dans une méthode de traitement du corps humain ou animal par thérapie.

**5.** Composé selon la revendication 1 pour utilisation dans une méthode de traitement d'un trouble lié au métabolisme.

**6.** Composé selon la revendication 1 pour utilisation dans une méthode de traitement du diabète de type I, diabète du type II, tolérance inadéquate au glucose, résistance à l'insuline, hyperglycémie, hyperlipidémie, hypertriglycéridémie, hypercholestérolémie, dyslipidémie ou syndrome X.

**7.** Composé selon la revendication 1 pour utilisation dans une méthode de traitement du diabète de type II.

**8.** Composé selon la revendication 1 pour utilisation dans une méthode de traitement de dyslipidémie.

**9.** Composé selon la revendication 1 pour utilisation dans une méthode de traitement de l'obésité.

**10.** Composé selon la revendication 1 pour utilisation dans une méthode de:

réduire la ration alimentaire d'un individu; ou
induire une satiété dans un individu; ou
contrôler ou diminuer le gain de poids d'un individu.

**11.** Utilisation d'un composé selon la revendication 1, pour la production d'un médicament pour le traitement d'un trouble lié au métabolisme.

**12.** Utilisation d'un composé selon la revendication 1 pour la production d'un médicament pour le traitement du diabète de type I, diabète de type II, tolérance inadéquate au glucose, résistance à l'insuline, hyperglycémie, hyperlipidémie, hypertriglycéridémie, hypercholestérolémie, dyslipidémie ou syndrome X.

**13.** Utilisation d'un composé selon la revendication 1 pour la production d'un médicament pour le traitement du diabète de type II.

**14.** Utilisation d'un composé selon la revendication 1 pour la production d'un médicament pour le traitement de la dyslipidémie.

**15.** Utilisation d'un composé selon la revendication 1 pour la production d'un médicament pour le traitement de l'obésité.

**16.** Utilisation d'un composé selon la revendication 1, pour la production d'un médicament pour:

réduire la ration alimentaire dans un individu; ou
induire une satiété dans un individu; ou
contrôler ou diminuer le gain de poids dans un individu.

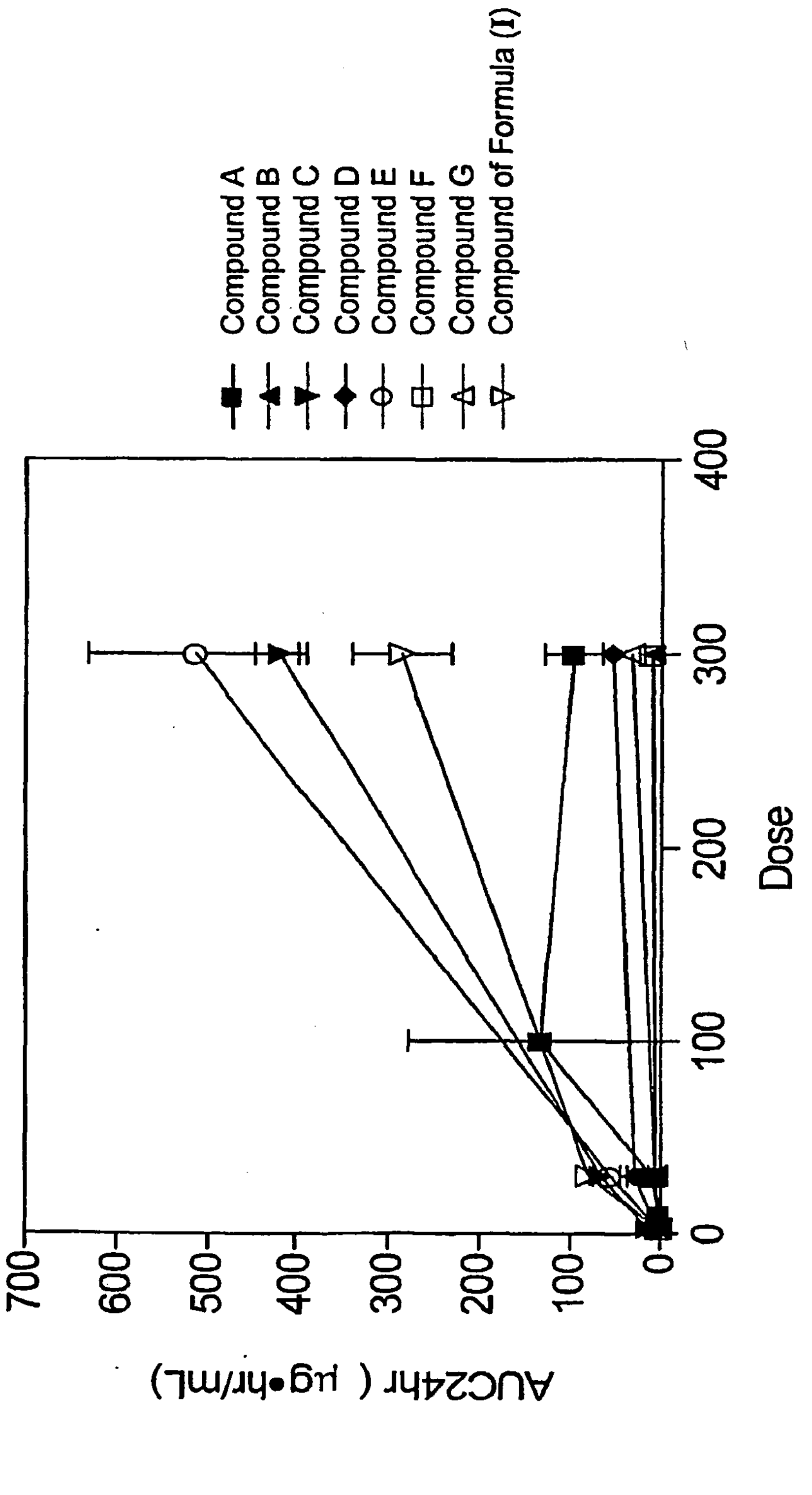
AUC24hr ( μg•hr/mL)
700
600
500
400
300
200
100
0
0
100
200
300
400
Dose
Compound A
Compound B
Compound C
Compound D
Compound E
Compound F
Compound G
Compound of Formula (I)

FIGURE 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005007647 A1
- WO 2005121121 A2
- WO 2005007647 A
- US 2004022417 W
- US 2004022327 W
- US 20041022327 W
- US 2004001267 W


### Non-patent literature cited in the description


- **Rotwein, R. et al.** *N. Engl. J. Med.,* 1983, vol. 308, 65-71
- **Rimoin, D. L.** Emery and Rimoin's Principles and Practice of Medical Genetics. 1996, vol. 1, 1401-1402
- **Le Stunff et al.** *Diabetes,* 1989, vol. 43, 696-702
- **Pederson, P.** *Diab. Metab. Rev.,* 1989, vol. 5, 505-509
- **Brancati, F. L. et al.** *Arch. Intern. Med.,* 1999, vol. 159, 957-963
- **Hill, J. O. et al.** *Science,* 1998, vol. 280, 1371-1374
- **Perry, I. J. et al.** *BMJ,* 1995, vol. 310, 560-564
- Remington, The Science and Practice of Pharmacy. Lippincott Williams & Wilkins, 2000
- **Reed ; Scribner.** *Diabetes, Obesity and Metabolism,* 1999, vol. 1, 75-86
- **Potenza, M. N. ; Lerner, M. R.** *Pigment Cell Research,* 1992, vol. 5, 372-378